# EUROPEAN PATENT SPECIFICATION

(11) **EP 4 103 179 B1**
(45) Date of publication and mention of the grant of the patent: **11.03.2026**
(21) Application number: 21753474.2
(22) Date of filing: 13.02.2021
(51) Int. Cl.: A61K 31/451, A61K 31/192, A61K 31/575, A61K 38/12, A61K 31/517, A61K 33/242, A61K 31/7016, A61K 31/437, A61K 31/519, A61K 39/00, A61P 21/00, A61P 25/28

(54) **COMBINATION THERAPY FOR USE IN TREATING AMYOTROPHIC LATERAL SCLEROSIS USING PRIDOPIDINE AND CNM-AU8 NANOCRYSTALLINE GOLD**
KOMBINATIONSTHERAPIE ZUR VERWENDUNG BEI DER BEHANDLUNG VON AMYOTROPHER LATERALSKLEROSE ENTHALTEND PRIDOPIDIN UND CNM-AU8 NANOKRISTALLINES GOLD
POLYTHÉRAPIE POUR LE TRAITEMENT DE LA SCLÉROSE LATÉRALE AMYOTROPHIQUE COMPRENANT DE LA PRIDOPIDINE ET DE L'OR NANOCRISTALLIN CNM-AU8

(30) Priority: 13.02.2020 US 202016789564; 21.10.2020 US 202017076069
(43) Date of publication of application: 21.12.2022
(73) Proprietor: Prilenia Neurotherapeutics Ltd., 6097200 Yakum (IL)
(72) Inventor: GEVA, Michal, 0000 Even-Yehuda (IL); HAYDEN, Michael, Yakum 6097200 (IL)
(74) Representative: Pearl Cohen UK
(86) International application number: PCT/IL2021/050172
(87) International publication number: WO 2021/161319

(56) References cited:
- WO-A1-2015/001379
- WO-A1-2018/191786
- WO-A1-2019/036358
- WO-A1-2019/036358
- WO-A2-2008/133884
- WO-A2-2014/181333
- IONESCU ARIEL ET AL: "Targeting the Sigma-1 Receptor via Pridopidine Ameliorates Central Features of ALS Pathology in a SOD1G93A Model", vol. 10, no. 210, 1 March 2019 (2019-03-01), pages 1 - 19, XP055847053, Retrieved from the Internet <URL:https://www.ncbi.nlm.nih.gov/pmc/articles/PMC6397200/pdf/41419_2019_Article_1451.pdf> DOI: 10.1038/s41419-019-1451-2
- GHEMRAWI ROSE ET AL: "Endoplasmic Reticulum Stress and Unfolded Protein Response in Neurodegenerative Diseases", INTERNATIONAL JOURNAL OF MOLECULAR SCIENCES, vol. 21, no. 17, 25 August 2020 (2020-08-25), Basel, CH, pages 6127, XP093245637, ISSN: 1422-0067, Retrieved from the Internet <URL:https://pubmed.ncbi.nlm.nih.gov/32854418/> DOI: 10.3390/ijms21176127
- IONESCU ARIEL, GRADUS TAL, ALTMAN TOPAZ, MAIMON ROY, SARAF AVRAHAM NOI, GEVA MICHAL, HAYDEN MICHAEL, PERLSON ERAN: "Targeting the Sigma-1 receptor via pridopidine ameliorates central features of ALS pathology in a SOD1G93A model", CELL DEATH DIS, vol. 10, no. 210, 1 March 2019 (2019-03-01), pages 1 - 19, XP055847053, DOI: 10.1038/s41419-019-1451-2
- FIGUEIREDO M: "FDA gives OK to HEALEY ALS platform trial testing zilucoplan, CNM- Au8, and verdiperstat", ALS NEWS TODAY, 27 January 2020 (2020-01-27), pages 1 - 6, XP055847069, Retrieved from the Internet <URL:https://alsnewstoday.com/news-posts/2020/01/27/fda-gives-ok-healey-als-platform-trial-testing-first-three-therapies-zilucoplan-cnm-au8-verdiperstat> [retrieved on 20210406]
- BIGICA A: "The road to success in ALS: Details of the AMX0035 CENTAUR trial", NEUROLOGYLIVE, 19 December 2019 (2019-12-19), pages 1 - 3, XP055847078, Retrieved from the Internet <URL:https://www.neurologylive.com/view/the-road-to-success-in-als-details-of-the-amx0035-centaur-trial> [retrieved on 20210406]
- BENDER K: "How an innovative trial design may change the ALS treatment landscape", NEUROLOGYLIVE, 26 February 2020 (2020-02-26), pages 1 - 6, XP055847083, Retrieved from the Internet <URL:https://www.neurologylive.com/view/how-an-innovative-trial-design-may-change-the-als-treatment-landscape> [retrieved on 20210406]

## Description

### FIELD OF THE INVENTION

Provided herein are compositions for use in treating a human subject afflicted with ALS. The compositions comprise a therapeutically effective amount of pridopidine or pharmaceutically acceptable salt thereof in combination with CNM-Au8 nanocrystalline gold as combination or add-on therapy.

### BACKGROUND OF THE INVENTION

Amyotrophic lateral sclerosis (ALS) is a devastating neurodegenerative disease characterized by progressive loss of motor neurons in the motor cortex, brainstem, and spinal cord (Peters 2015). This rapidly progressing fatal disease leads to weakness of limb, respiratory, and bulbar muscles. Patients progressively lose control of voluntary muscles, leading to loss of limb function and the ability to chew, swallow, speak and eventually breathe.

ALS is a rare condition, having a mean incidence rate of 2.8/100,000 in Europe and 1.8/100,000 in North America, and a mean prevalence rate of 5.40/100,000 in Europe and 3.40/100,000 in North America (Bozzoni 2016).

In ~90% of cases, there is no apparent genetic mutation underling the disease (sporadic ALS). Approximately 5% to 10% of ALS cases are familial (fALS) and caused by a genetic mutation. There are >25 genes which are recognized as associated and causative of ALS. The most common disease-causing mutations are C9ORF72 (33.7% of fALS) and SOD1 (14.8% of fALS) (Zou et al. 2017).

ALS patients experience signs and symptoms of progressive muscle atrophy and weakness, increased fatigue, and problems with swallowing, which typically lead to aspiration pneumonia, respiratory failure, and death. Progressive functional deficits lead to an overall loss of independence and death.

ALS begins in the limbs in about two-thirds of patients, most often in the arms. The first symptoms are usually unilateral and focal. Early findings include foot drop, difficulty walking, loss of hand dexterity or difficulty lifting the arms over the head. Eventually, limb function can be lost, leading to dependence on caregivers. Patients may fall or lose the ability to walk all-together. Bulbar-onset disease, often occurring in older women, appears to have a worse prognosis (Chiò et al. 2009).

Non-motor symptoms of ALS include behavioral disturbances, dysexecutive impairment, and frontotemporal dementia. Overt frontotemporal dementia occurs in approximately 15% of patients, but as many as 50% are impaired by neuropsychological tests (Lomen-Hoerth et al. 2003; Gordon et al. 2011). Changes involve language, judgment, personality, affect and executive function. Patients with ALS and dementia have shorter survival, possibly as a result of poor decision-making ability (Olney et al. 2005). The median survival is 2 to 4 years from onset; only 5-10% of patients survive beyond 10 years (Chiò et al. 2013).

There is currently no known disease-modifying therapy for the treatment of ALS. Two approved drugs are riluzole and edatavone. Riluzole slows the rate of progression of the disease and prolongs survival by 2 or 3 months and edaravone slows physical decline (Jaiswal 2018).

The pathophysiological mechanism of ALS appears to be multifactorial, and several mechanisms contribute to neurodegeneration. These include impaired autophagy, mitochondrial dysfunction, glutamate excitotoxicity, oxidative stress and neuroinflammation. Early pathological events in ALS include perturbations in axonal transport, formation of toxic protein aggregates and NMJ disruption. All of these lead to axonal degeneration and motor neuron death (Ionescu et al. 2019).

The neuropathological features of ALS include muscle atrophy, loss of anterior horn cells, and sclerosis of the spinal cord lateral columns (Martel 2016). Gliosis, defined as activation of astrocytes and microglia, is also a hallmark of ALS.

Pridopidine (formerly ACR16, Huntexil^{®}) is a unique compound in clinical development for the treatment of Huntington's disease (HD) and ALS. The chemical name of pridopidine is 4-(3-(Methylsulfonyl)phenyl)-1-propylpiperidine, and its Chemical Registry Number is CAS 346688-38-8 (CSID:7971505, 2016). The Chemical Registry number of pridopidine hydrochloride is 882737-42-0 (CSID:25948790 2016). Processes of synthesis of pridopidine and a pharmaceutically acceptable salt thereof are disclosed in U.S. Patent No. 7,923,459 and PCT Application Publication No. WO 2017/015609. U.S. Patent No. RE46,117 discloses pridopidine for the treatment of a variety of diseases and disorders.

Pridopidine is a highly potent and selective Sigma-1 receptor (S1R) agonist developed by Prilenia for the treatment of neurodegenerative and neurodevelopmental disorders.

The S1R is an intracellular ligand-operated protein located mainly at the Mitochondria Associated Membranes (MAM) and is implicated in cellular differentiation, neuroplasticity, neuroprotection and cognitive function in the brain.

The S1R is widely expressed in the nervous system (Gundlach et al., 1986) with high expression in motor neurons of the brainstem and spinal cord (Mavlyutov et al. 2010; 2012; Waterhouse et al. 1997).

The S1R regulates key cellular processes relevant to neurodegenerative diseases, such as calcium signaling, cytoskeleton dynamics, neurotrophic factor release, mitochondrial function and ER stress (Ryskamp, Korban, et al. 2019; Hayashi 2019; Kourrich et al. 2012; Su et al. 2010). These pathways are impaired in ALS.

Pridopidine exerts neuroprotective properties via activation of the S1R in numerous nonclinical models including HD, ALS, Parkinson's disease (PD) and Alzheimer's (AD). Pridopidine enhances secretion of the neuroprotective brain-derived neurotrophic factor (BDNF) in a neuroblastoma cell line, in a S1R-dependent manner (Geva 2016) and rescues spine impairment and aberrant calcium signaling by activation of the S1R (Ryskamp 2017).

PCT International Patent Application Publication No. WO2016/138135 discloses use of S1R agonists to treat, inter alia, familial adult amyotrophic lateral sclerosis (ALS) and juvenile amyotrophic lateral sclerosis (ALS).

WO 2019/036358 discloses a method for treating a human subject afflicted with ALS by administering a therapeutically effective amount of pridopidine as monotherapy or together with riluzole, edaravone or dextromethorphan/quinidine as combination or add-on therapy.

Ionescu et al. disclose that targeting the sigma-1 receptor via pridopidine ameliorates central features of ALS pathology in a SOD1G93A mouse model (Cell Death and Disease, 2019, 10: 210; https://doi.org/10.1038/s41419-019-1451-2).

There remains an unmet need to provide effective treatments for ALS, in particular sporadic ALS.

### SUMMARY OF THE INVENTION

The present invention provides a combination of a composition comprising pridopidine or pharmaceutically acceptable salt thereof, and a second composition comprising CNM-Au8 nanocrystalline gold, for use in treating amyotrophic lateral sclerosis (ALS) in a subject.

In some embodiments, the ALS is sporadic ALS.

In some embodiments, the ALS is familial ALS.

In an embodiment, this combination is effective to delay the onset of a symptom or slow the progression of a symptom or improve a symptom of ALS in a subject.

In an embodiment, the symptom is muscle stiffness, muscle weakness, muscle wasting, muscle cramps, difficulty speaking, difficulty swallowing, difficulty breathing, difficulty chewing, difficulty walking, fasciculations, worsening posture, respiratory function, muscle strength, bulbar function, speech, salivation, difficulty swallowing, difficulty in handwriting, difficulty in cutting food and handling utensils, difficulty in dressing, dyspnea, orthopnea, or combination thereof.

In an embodiment, the combination is effective to enhance BDNF axonal transport in motor neurons, enhance ERK activation in motor neurons, improve NMJ formation and preservation, preserve NMJ structure, enhance NMJ function, improve innervation rate of muscle tissue, enhance motor neuron axonal growth, reduce axonal degeneration, reduce motor neuron axonal degeneration, enhance muscle cell survival, enhance muscle fiber diameter and function or reduce SOD1 aggregation.

In an embodiment, the combination further lessens pseudobulbar disease progression, reduces progression of muscle fiber wasting, and/or improves muscle contraction in the subject afflicted with ALS.

In an embodiment, the composition comprising pridopidine or pharmaceutically acceptable salt thereof and the second composition are administered daily, twice daily, twice a week, three times a week or more often than once daily.

In another embodiment, the composition comprising pridopidine or pharmaceutically acceptable salt thereof is administered orally or by gastric tube.

In another embodiment, the amount of pridopidine or pharmaceutically acceptable salt thereof administered is 10 mg/day to 135 mg/day.

In another embodiment, the pridopidine is pridopidine hydrochloride.

In some embodiments, the amount of CNM-Au8 nanocrystalline gold administered is between 5-50 mg/day (oral).

In some embodiments, the administration of the second composition precedes the administration of pridopidine or pharmaceutically acceptable salt thereof. In other embodiments, the administration of the composition comprising pridopidine or pharmaceutically acceptable salt thereof precedes the administration of the second composition. In other embodiments, the composition comprising pridopidine or pharmaceutically acceptable salt thereof is administered adjunctively to the second composition. In other embodiments, the second composition is administered adjunctively to the composition comprising pridopidine or pharmaceutically acceptable salt thereof.

### BRIEF DESCRIPTION OF THE FIGURES

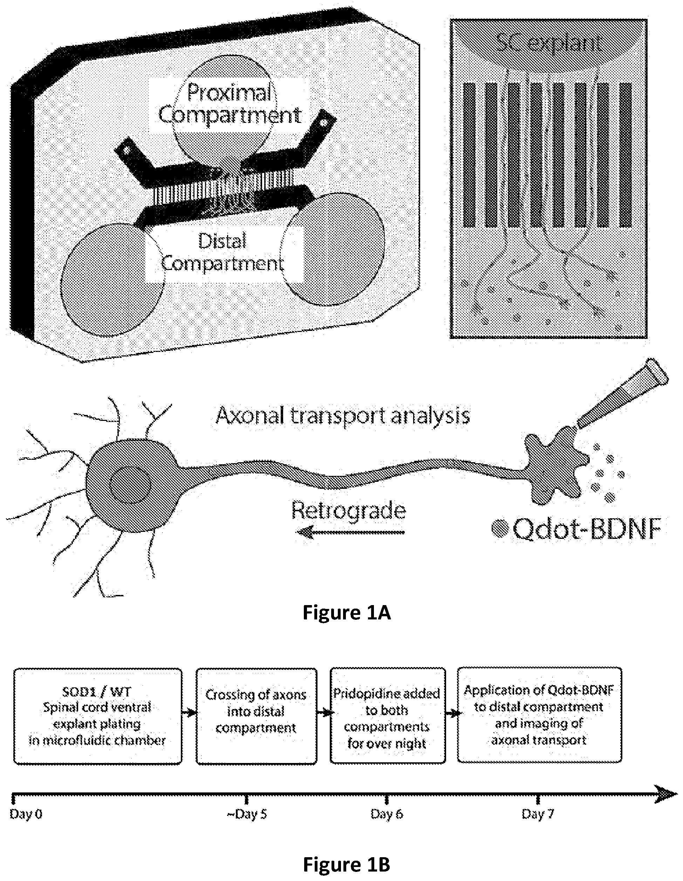

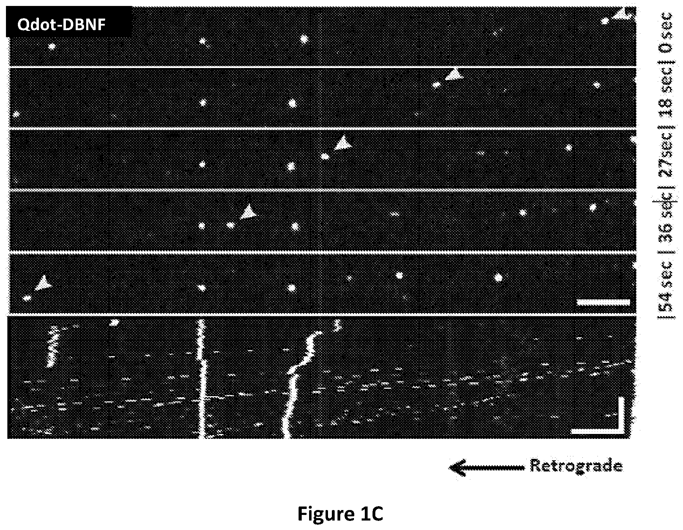

**Figures 1A-1C****.** Axonal transport assay. **Figure 1A****.** Schematic illustration of the experimental system (microfluidic chamber) for axonal transport tracking in motor neurons (MNs). **Figure 1B****.** Experimental workflow for the axonal transport assay. **Figure 1C****.** Time lapse images and kymograph of Qdot-BDNF axonal transport in MN axon.

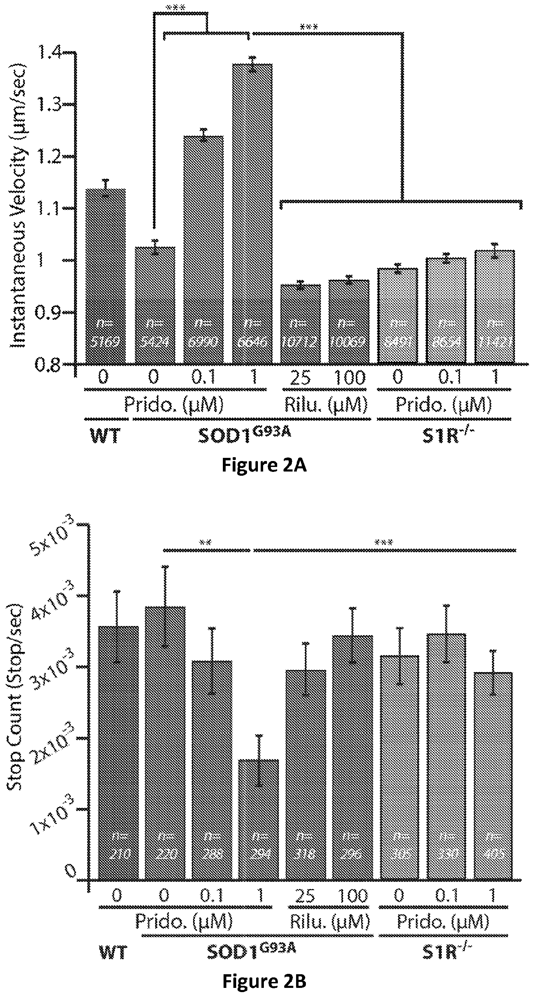

**Figures 2A-2B****.** Graphs showing effect of pridopidine on instantaneous velocity values and particle stop count of Qdot-BDNF along the axon **Figure 2A****:** Pridopidine's effect on instantaneous velocity values (µm/sec) for Qdot- BDNF particles in WT, SOD1G93A or Sigma-1 receptor knock out (S1R-/-) MNs. Pridopidine increases instantaneous velocity of Qdot-BDNF in SOD1 G93A MNs in a SIR-dependent mechanism. **Figure 2B****:** Pridopidine's effect on particle stop count (number of counted stops of Qdot-BDNF per second). Pridopidine reduces stop count on Qdot-BDNF in SOD1 G93A MNs in a S1R-dependent mechanism. Data are shown as the mean ± SEM. **p value < 0.01; ***p value < 0.001 (n =6 independent experiments; the sample size for each experiment is indicated on bars; Student's t test)

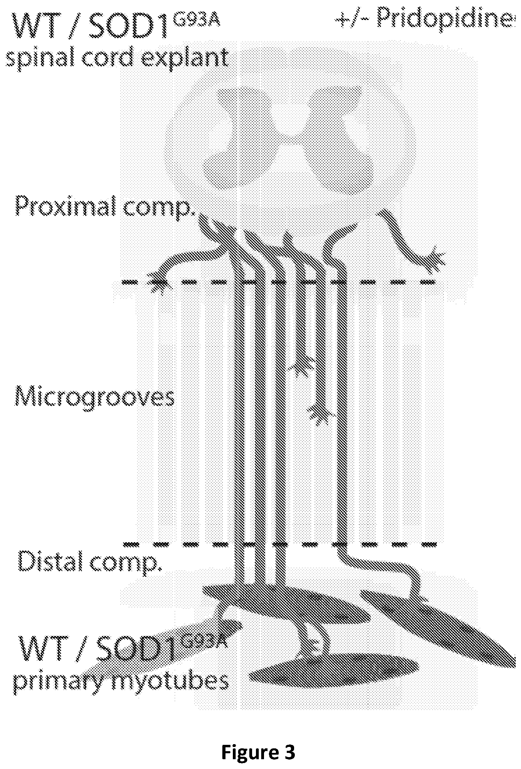

**Figure 3****.** Schematic illustration of the experimental procedure for neuromuscular coculture assays measuring muscle innervation and Neuro Muscular Junction function (NMJ). Spinal cord explant is cultured in the proximal compartment and primary myocytes are cultured in the distal compartment. Pridopidine is added to both proximal and distal compartments.

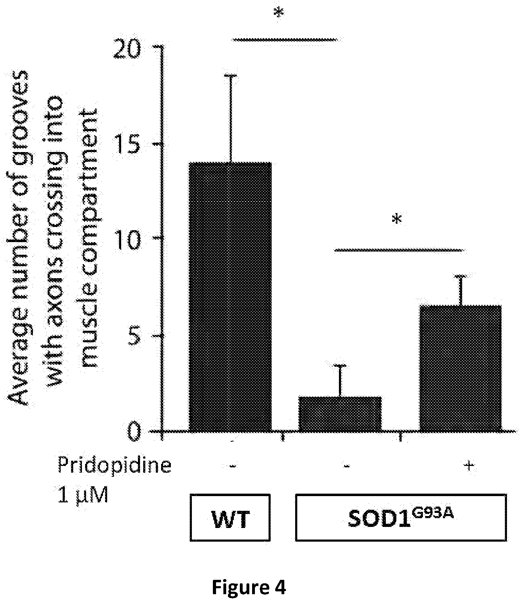

**Figure 4****.** Graph showing pridopidine's effect on axonal growth in SOD1G93A MNs, which measures the number of grooves with axons crossing into the muscle compartment. Pridopidine increases axonal growth in SOD1 G93A MNs. Data are shown as the mean ± SEM. *p value < 0.05.

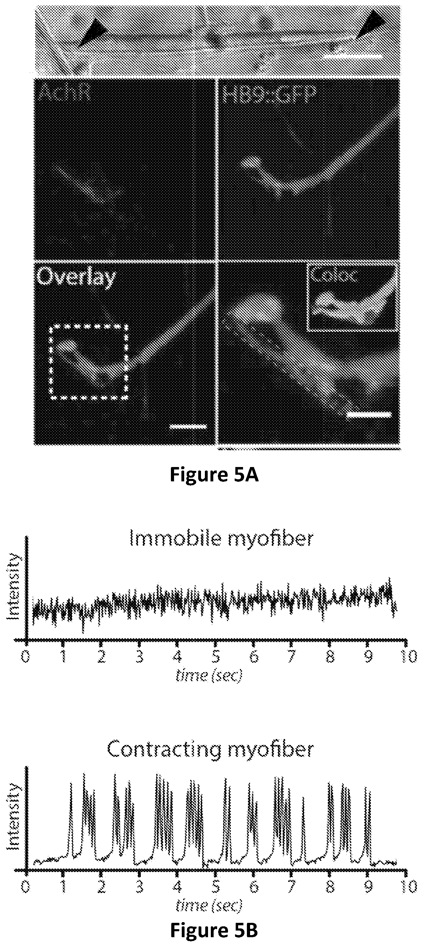

**Figures 5A - 5B****.** Results of Micro Fluidic Compartment (MFC) co-culture assays **Figure 5A****:** Microscope image of neuromuscular junction in microfluidic co-culture chamber. Upper panel: Phase image of a myocyte in the distal compartment connected by axons (arrowheads). Lower panel: High magnification images of myocyte:MN contact points. The muscle compartment is visualized by fluorescently marking the acetyl choline receptor (AchR) with fluorescently-labelled bungarotoxin (Btx). The neuronal compartment is marked by expressing green fluorescent protein (GFP) on the MN-specific genetic marker HB9. (Hb9:GFP) Inset: rendering of colocalization. **Figure 5B****:** Muscle contraction traces as extracted from intensity over time measurements of muscle contraction in microfluidic co-culture chamber.

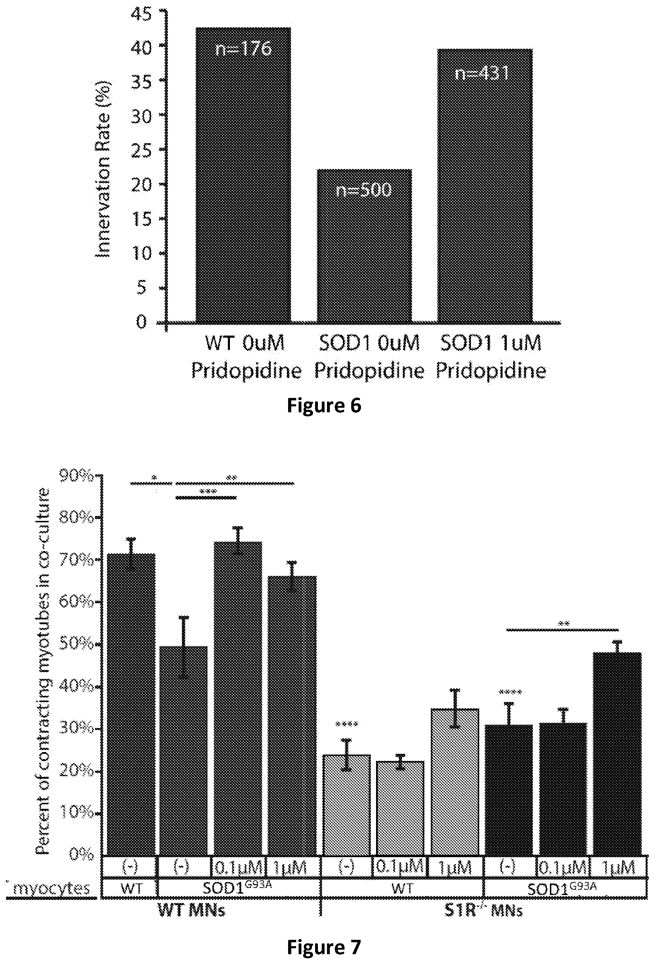

**Figure 6****.** Graph showing pridopidine's effect on innervation rate in WT and SOD1G93A (SOD1) ALS myocytes. Pridopidine increases innervation rate in SOD1 G93A MN-muscle co-culture.
**Figures 7****.** Graphs showing level of contracting myocytes in MN-myocyte co-culture. SOD1 G93A myocytes show reduced contractility. Similarly, both WT and SOD1 myocytes innervated with SIR-/- neurons show reduced contractility. Pridopidine's effect on neuromuscular junction (NMJ) in muscle tissue from WT or SOD1G93A and motor neurons from WT or S1R-/- mice. Pridopidine restores muscle contractility in SOD1 G93A muscles in a S1R-mediated mechanism. *p value <0.05; **p value < 0.01, ***p value < 0.001, ****p value < 0.0001. (n = number of microfluidic chambers from 3 or more independent experiments; Student's t test)

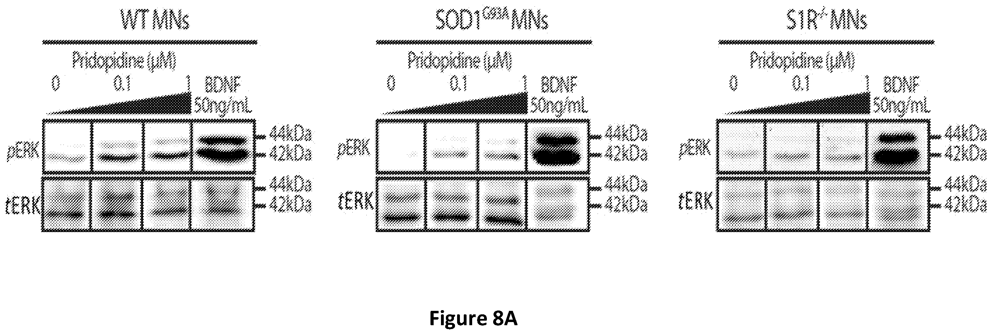

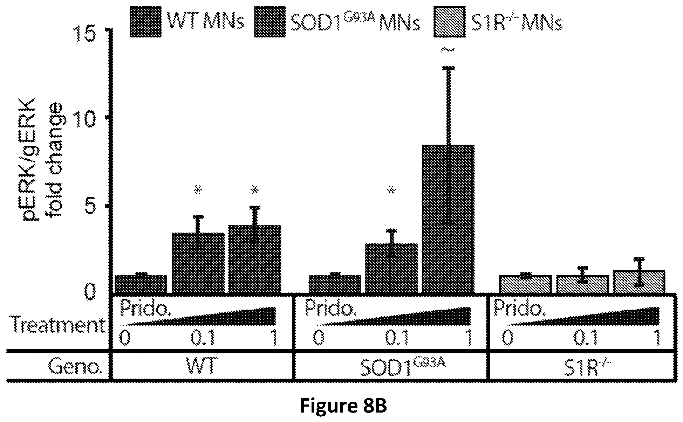

**Figures 8A-8B****.** Effect of pridopidine on ERK levels. **Figure 8A****:** Pridopidine's effect on total ERK (tERK) and phosphorylated ERK (pERK) levels shown in Western blots from WT, SODIG93A, and S1R-/- MNs culture extracts. Pridopidine increases pERK in a dose-dependent and S1R dependent manner. **Figure 8B****:** Quantification of pridopidine's effect of ERK activation as measured by tERK/pERK. Data are shown as the mean pERK/ERK± SEM. Pridopidine increases pERK in a S1R-mediated mechanism. *p value < 0.05, ~p value < 0.1 (n = 3 independent experiments; Student's t test.)

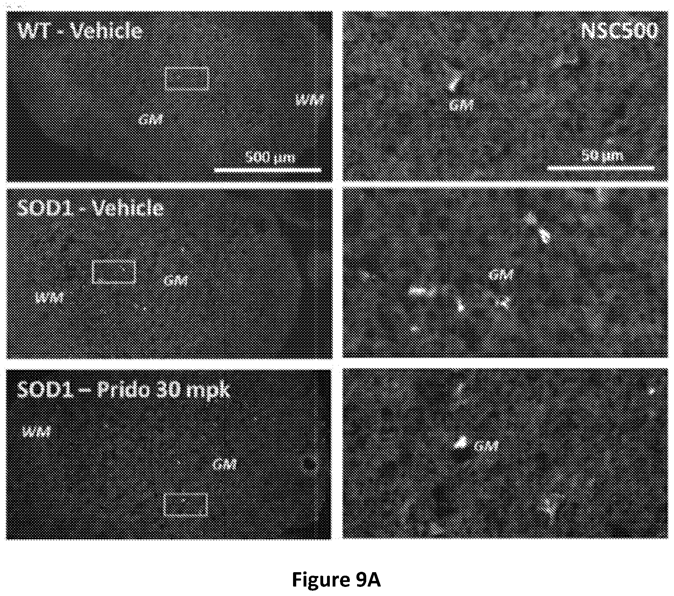

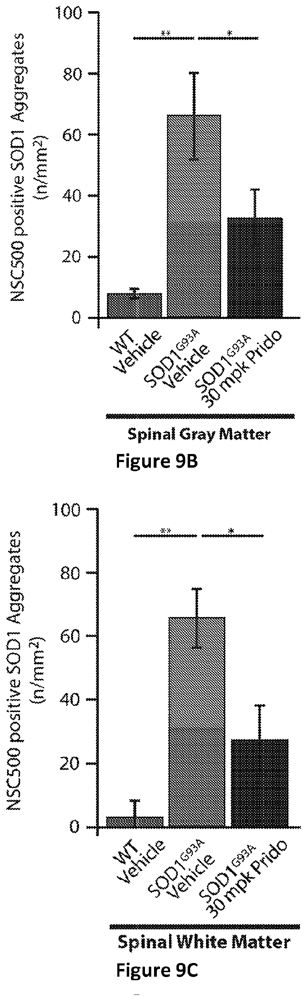

**Figures 9A-9C****.** Effect of pridopidine on SOD1 aggregates. **Figure 9A****:** Visualization and quantification of fluorescently labeled spinal cords with the NSC500 dye to label mutant SOD1 aggregates in WT and SOD1G93A spinal cords, treated or not with pridopidine 30 mg/kg. Pridopidine reduces SOD1 aggregation in SOD1 G93A muscle. **Figure 9B****:** Quantitative analysis of the number of aggregates per area identified in the gray matter (GM). Pridopidine reduces SOD1 aggregation in spinal grey matter. **Figure 9C****:** Quantitative analysis of the number of aggregates per area identified in the white matter (WM). Data are shown as the mean ± SEM. Pridopidine reduces SOD1 aggregation in spinal white matter *p value < 0.05; **p value < 0.01 (n =4 mice in each group; one-way ANOVA followed by Fisher's LSD post hoc tests.)

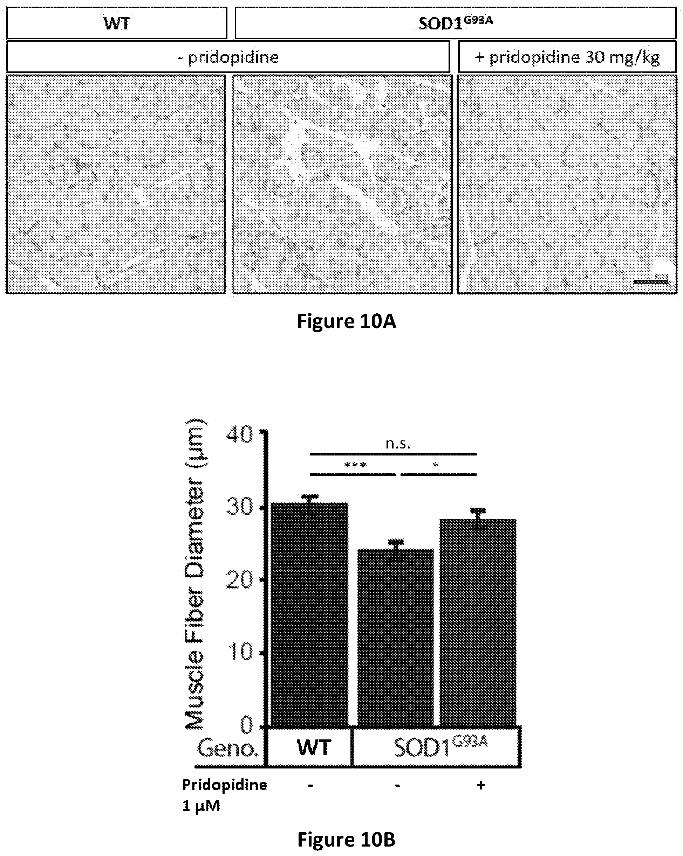

**Figures 10A-10B****.** Effect of pridopidine on muscle tissue. **Figure 10A****:** Representative images of hematoxylin and eosin (H&E)-stained cross sections from Gastrocnemius muscle of WT or SOD1G93A mice treated or not with Pridopidine 30mg/kg. Pridopidine rescues muscle fiber wasting in SOD1 G93A **muscles.** **Figure 10B****:** Assessment of pridopidine's effect on muscle fiber wasting: quantitative analysis of pridopidine's effect on muscle fiber diameter. Pridopidine rescues muscle fiber wasting in SOD1 G93A muscles. Data are shown as mean ± SEM (n = number of NMJs). *p value < 0.05; ***p value < 0.001 (n = 5 mice in each group; Student's t test)

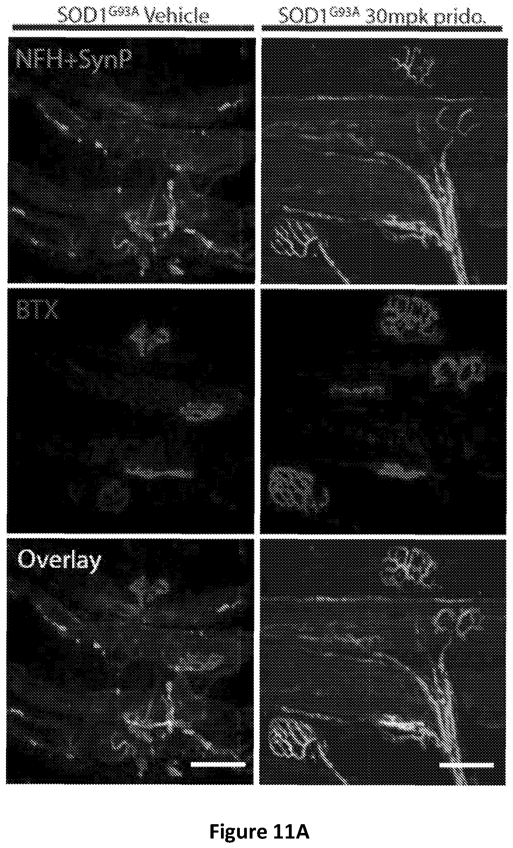

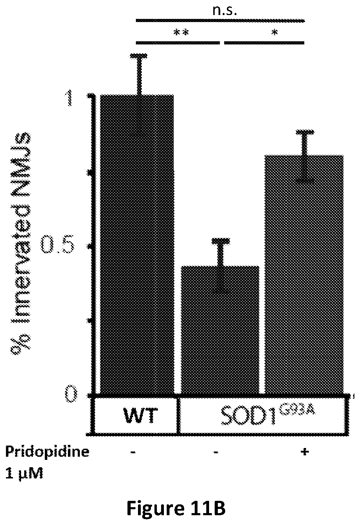

**Figures 11A-11B****.** Effect of pridopidine on NMJ. **Figure 11A****:** Pridopidine's effect on NMJ preservation in vivo. The muscle compartment is marked by fluorescently labelled Btx. The neuronal compartment is marked by immunostaining of neurofilament heavy (NFH) and synaptophysin (SynP) proteins. Bottom panel: An overlay of the neuronal and muscular markers shows an overlap at NMJ sites. Pridopidine preserves NMJ numbers in SOD1 G93A muscle. **Figure 11B****:** Quantitative analysis of pridopidine's effect on the percentage of innervated NMJs. Data are shown as mean ± SEM (n = number of NMJs). Pridopidine prevents loss of NMJs in SOD1 G93A muscles. *p value < 0.05; **p value < 0.01; (n = 5 mice in each group; Student's t test).

### DETAILED DESCRIPTION OF THE INVENTION

The invention provides a combination of a composition comprising pridopidine or pharmaceutically acceptable salt thereof, and a second composition comprising CNM-Au8 nanocrystalline gold, for use in treating amyotrophic lateral sclerosis (ALS) in a subject.

In an embodiment, the combination of the pridopidine or pharmaceutically acceptable salt thereof and the CNM-Au8 nanocrystalline gold is effective to delay the onset, improve, or lessen the decline of ALS functionality, respiratory function, muscle strength, bulbar function, speech, or any combination thereof in a subject afflicted with amyotrophic lateral sclerosis (ALS).

In an embodiment, the ALS is sporadic ALS.

In an embodiment, the ALS is familial ALS (FALS). In some embodiments the ALS is juvenile ALS (JALS).

In some embodiments the ALS is not FALS. In some embodiments the ALS is not juvenile ALS (JALS).

In an embodiment, the type of ALS is classic, bulbar, flail arm, flail leg, pyramidal and respiratory ALS, progressive muscular atrophy, primary lateral sclerosis, or progressive bulbar palsy.

In an embodiment, the subject carries a mutant version of a gene that causes or contributes to ALS pathogenesis. In some embodiments the mutant version is of a gene selected from superoxide dismutase 1 (SOD1), TAR DNA-binding protein (TARDBP) encoding TDP-43, fused in sarcoma (FUS), p62 (SQSTM1), ubiliquin-2 (UBQLN2), TANK-binding kinase 1 (TBK1), profilin 1 (PFN1), VCP or p97 (VCP), angiogenin (ANG), optineurin (OPTN), C9orf72, Sigma-1 Receptor (S1R), Tubulin alpha-4A (TUBA4A), Dynactin (DCTN1), , hnRNPA1 (HNRNPA1), Matrin 3(MATR3), Coiled-coil-helix-coiled-coil-helix domain containing 10 (CHCHD10), and any combination thereof.

In some embodiments, maintaining, improving, or lessening the decline of ALS functionality comprises maintaining, improving, or lessening the decline of speech, salivation, swallowing, handwriting, cutting food, and handling utensils, dressing and hygiene, turning in bed and adjusting bed clothes, walking, climbing stairs, dyspnea, orthopnea, respiratory insufficiency, or any combination thereof in ALS patients.

In an embodiment, the change in respiratory function is assessed by slow vital capacity (SVC).

In an embodiment, the maintaining, improving, or lessening the decline in muscle strength is measured isometrically using hand-held dynamometry (HHD), bilateral Hand Grip or combination thereof.

In an embodiment, the maintaining, improving, or lessening the decline in bulbar function is measured by the ALSFRS-R bulbar subdomain (Q1-Q3) score.

In an embodiment, the maintaining, improving, or lessening the decline in bulbar function is measured by the CNS-BFS.

In an embodiment, the subject has bulbar dysfunction.

In an embodiment, the subject has rapid pre-baseline progression.

In an embodiment, the amount of pridopidine is effective to change time to first evidence of bulbar dysfunction.

In an embodiment, the maintaining, improving, or lessening the decline in speech is measured by the ALSFRS-R speech domain score (Q1).

In an embodiment, the maintaining, improving, or lessening the decline in speech is measured by automated algorithmic assessment of speech collected digitally to detect early changes and tracking progression. In an embodiment of the invention, the maintaining, improving, or lessening the decline is measured by the ALS Functional Rating Scale-Revised (ALSFRS-R).

In an embodiment, the amount of pridopidine or pharmaceutically acceptable salt thereof in combination with CNM-Au8 nanocrystalline gold is effective to inhibit or reduce progression of a symptom of ALS in the subject.

In an embodiment, the symptom of ALS is a clinical symptom of ALS.

In an embodiment, the symptom of ALS is muscle weakness and hypotrophy, fasciculations and cramps, spastic hypertonus, hyperreflexia, dysarthria, dysphagia and respiratory weakness, behavioral disturbances, dysexecutive impairment, functionality, respiratory function, muscle strength, bulbar function, speech, or any combination thereof or frontotemporal dementia.

In an embodiment, the symptom of ALS is a neuropathological symptom.

In some embodiments, the symptom is bulbar palsy or pseudobulbar affect (PBA).

In an embodiment, the symptom of ALS is muscle atrophy, loss of motor neurons, loss of anterior horn cells, sclerosis of the spinal cord lateral columns, or gliosis.

In one embodiment, the symptom of ALS is a rate of decline (a) in pulmonary function, (b) in functional disability, or (c) in the ability score for the lower extremities. In an embodiment, the amount of pridopidine is effective to increase survival of the subject or cause neuroprotection in the subject.

In some embodiments of the invention, treatment of the subject with pridopidine or pharmaceutically acceptable salt thereof in combination with CNM-Au8 nanocrystalline gold results in a delay of onset of an ALS symptom, lessened decline of an ALS symptom or an improvement of an ALS symptom in the subject, in one or more of the following domains, 1) speech, 2) salivation, 3) swallowing, 4) handwriting, 5) cutting food and handling utensils (with or without gastrostomy), 6) dressing and hygiene, 7) turning in bed and adjusting bed clothes, 8) walking, 9) climbing stairs, 10) breathing, 11) dyspnea, 12) orthopnea, and 13) insufficiency.

In some embodiments, patients are monitored for changes in the above domains using a rating scale, for example the Amyotrophic Lateral Sclerosis Functional Rating Scale (ALSFRS) or revised ALSFRS (ALSFRS-R) and a functional change in a patient is monitored over time.

In some embodiments, pseudobulbar affect (PBA) (as measured by CNS-LS) is monitored in the patients. In some embodiments, the severity and /or frequency of emotional outbursts in subjects experiencing PBA is reduced with pridopidine treatment.

In some embodiments, use of pridopidine or pharmaceutically acceptable salt thereof in combination with CNM-Au8 nanocrystalline gold maintains, improves or lessens the decline of in disease severity as measured by the ALS Functional Rating Scale-Revised (ALSFRS-R) in ALS patients and/or ALSAQ-5.

In some embodiments, use of pridopidine or pharmaceutically acceptable salt thereof in combination with CNM-Au8 nanocrystalline gold maintains, improves, or lessens the decline in respiratory function as assessed by slow vital capacity (SVC) in ALS patients.

In some embodiments, use of pridopidine or pharmaceutically acceptable salt thereof in combination with CNM-Au8 nanocrystalline gold maintains, improves, or lessens the decline in muscle strength as measured by handheld dynamometry (HHD) in ALS patients.

In some embodiments, use of pridopidine or pharmaceutically acceptable salt thereof in combination with CNM-Au8 nanocrystalline gold results in maintenance, reduction or less increase in phosphorylated neurofilament heavy chain (pNfH) and neurofilament light chain (NfL) in plasma and CSF in ALS patients.

In some embodiments, use of pridopidine or pharmaceutically acceptable salt thereof in combination with CNM-Au8 nanocrystalline gold results in maintenance, reduction or less increase in urinary neurotrophin receptor p75 extracellular domain (p75^{ECD}) in ALS patients.

Several studies have found that speech features, such as jitter, shimmer, articulatory rate, speaking rate, and pause rate, are affected in ALS. In some embodiments of the invention, use of pridopidine maintains, improves, or lessens the decline in speech characteristics as measured by automated algorithmic assessment of speech collected digitally as described in Stegmann, G. et al., 2020.

In some embodiments, use of pridopidine or pharmaceutically acceptable salt thereof in combination with CNM-Au8 nanocrystalline gold maintains, improves, lessens the decline, delays of onset in speech characteristics as measured by the slope of change in the CNS-BFS speech subdomain in ALS patients.

In some embodiments, use of pridopidine or pharmaceutically acceptable salt thereof in combination with CNM-Au8 nanocrystalline gold in ALS patients maintains, improves, delays the onset or lessens the decline in voice characteristics as determined by Aural Analytics set of analyses in ALS patients.

In some embodiments, use of pridopidine or pharmaceutically acceptable salt thereof in combination with CNM-Au8 nanocrystalline gold maintains, improves, delays the onset or lessens the decline in cognitive function as measured by the Edinburgh Cognitive and Behavioral ALS Screen (ECAS) in ALS patients.

In some embodiments, use of pridopidine or pharmaceutically acceptable salt thereof in combination with, CNM-Au8 nanocrystalline gold maintains, improves, delays the onset, or lessens the decline in home-based clinical assessments (weekly ALSFRS-R, SVC, pinch strength) in ALS patients.

In some embodiment, use of pridopidine or pharmaceutically acceptable salt thereof in combination with CNM-Au8 nanocrystalline gold maintains, improves, delays the onset or lessens the decline in bulbar function as measured by the CNS-BFS (Center for Neurologic Study Bulbar Function Scale) and the bulbar sub-domain (Q1-Q3) score of the ALSFRS-R total score in ALS patients.

In some embodiment, use of pridopidine or pharmaceutically acceptable salt thereof in combination with CNM-Au8 nanocrystalline gold maintains, improves, delays the onset, or lessens the decline in muscle strength, as measured isometrically using hand-held dynamometry (HHD) and grip strength in ALS patients.

In some embodiment, use of pridopidine or pharmaceutically acceptable salt thereof in combination with CNM-Au8 nanocrystalline gold maintains, improves, delays the onset, or lessens the decline in bulbar function as measured by the slope of change in the CNS-BFS total score in ALS patients.

In some embodiment, use of pridopidine or pharmaceutically acceptable salt thereof in combination with CNM-Au8 nanocrystalline gold maintains, improves, delays the onset, or lessens the decline in bulbar function as measured by the slope of change in the CNS-BFS total score in ALS patients whose calculated ALSFRS-R slope at baseline (48-ALSFRS-R total score at baseline/time since onset) is equal to or greater than 0.75 pt/month.

In some embodiment, use of pridopidine or pharmaceutically acceptable salt thereof in combination with CNM-Au8 nanocrystalline gold reduces the percentage of ALS patients who develop bulbar symptoms by 6 months among participants without bulbar symptoms at baseline (as defined as a CNS-BFS score < 30 at baseline) in the active compared to placebo groups.

In an embodiment, the pridopidine or pharmaceutically acceptable salt thereof in combination with CNM-Au8 nanocrystalline gold is administered daily, or is administered more often than once daily, or is administered thrice daily.

In an embodiment, the pridopidine or pharmaceutically acceptable salt thereof in combination with CNM-Au8 nanocrystalline gold is administered less often than once daily, for example, on alternate days, three times per week, twice per week or once per week.

In an embodiment, the pridopidine or pharmaceutically acceptable salt thereof in combination with CNM-Au8 nanocrystalline gold is administered daily, twice a week, three times a week or more often than once daily.

In an embodiment, the pridopidine or pharmaceutically acceptable salt thereof in combination with CNM-Au8 nanocrystalline gold is administered orally.

In some embodiments, a unit dose of the pharmaceutical composition contains 10-250 mg pridopidine or pharmaceutically acceptable salt thereof. In some embodiments the composition comprises 10 mg, 22.5 mg, 45 mg, 67.5 mg, 90 mg, or 112.5 mg of pridopidine.

In an embodiment, between 10 - 250 mg pridopidine or pharmaceutically acceptable salt thereof is administered to the patient per day. In another embodiment, between 45-180 mg pridopidine or pharmaceutically acceptable salt thereof is administered to the patient per day. In another embodiment, 10 mg, 22.5 mg, 45 mg, 67.5, mg, 90 mg, 100 mg, 112.5 mg, 125 mg, 135 mg, 150 mg, or 180 mg pridopidine or pharmaceutically acceptable salt thereof is administered to the patient per day.

In an embodiment, the pharmaceutical composition is administered twice per day. In another embodiment, an equal amount of the pharmaceutical composition is administered at each administration. In an embodiment, the two doses are administered at least 6 hours apart, at least 7 hours, at least 8 hours, at least 9 hours, at least 10 hours, at least 11 hours apart. In some embodiments, the pharmaceutical composition is administered for at least 12 weeks, at least 20 weeks, at least 24 weeks, at least 26 weeks, at least 52 weeks, or at least 78 weeks.

In an embodiment of the invention, the pridopidine salt is selected from the group consisting of hydrochloride, hydrobromide, nitrate, perchlorate, phosphate, sulphate, formate, acetate, aconate, ascorbate, benzenesulphonate, benzoate, cinnamate, citrate, the embonate, enantate, fumarate, glutamate, glycolate, lactate, maleate, malonate, mandelate, methane sulphonate, naphthalene-2-sulphonate, phthalate, salicylate, sorbate, stearate, succinate, tartrate and toluene-p-sulphonate: In an embodiment, the pridopidine salt is pridopidine hydrochloride.

In an embodiment of the invention, the subject is a human subject.

In an embodiment, the pharmaceutical composition for use in treating a subject afflicted with ALS comprises pridopidine or pharmaceutically acceptable salt thereof and a second composition comprising CNM-Au8 nanocrystalline gold are prepared to be administered simultaneously or contemporaneously.

In an embodiment, the pharmaceutical composition is in a unit dosage form, useful in treating subject afflicted with ALS, which comprises:
a) an amount of pridopidine or a pharmaceutically acceptable salt thereof;
b) an amount of CNM-Au8 nanocrystalline gold,
wherein the respective amounts of said CNM-Au8 nanocrystalline gold and said pridopidine or pharmaceutically acceptable salt thereof in said composition are effective, upon concomitant administration to said subject of one or more of said unit dosage forms of said composition, to treat the subject.

In an embodiment, the pharmaceutical composition comprises an amount of pridopidine for use in treating a subject afflicted with ALS as an add-on therapy to a second compound which is CNM-Au8 nanocrystalline gold.

In an embodiment, the pharmaceutical composition comprises an amount of pridopidine or pharmaceutically acceptable salt thereof for use in treating a subject afflicted with ALS simultaneously or contemporaneously with a second compound which is CNM-Au8 nanocrystalline gold.

In another embodiment, the pharmaceutical composition comprises an amount of CNM-Au8 nanocrystalline gold, for use in treating a subject afflicted with ALS as an add-on therapy to pridopidine or pharmaceutically acceptable salt thereof.

In another embodiment the pharmaceutical composition comprises an amount of CNM-Au8 nanocrystalline gold for use in treating a subject afflicted with ALS simultaneously or contemporaneously with pridopidine or pharmaceutically acceptable salt thereof.

The invention also provides CNM-Au8 nanocrystalline gold for use as an add-on therapy to pridopidine or pharmaceutically acceptable salt thereof in treating a subject afflicted with ALS.

The invention also provides for pridopidine or pharmaceutically acceptable salt thereof for use as an add-on therapy to CNM-Au8 nanocrystalline gold in treating a subject afflicted with ALS.

The invention also provides for a combination of CNM-Au8 nanocrystalline gold and pridopidine or pharmaceutically acceptable salt thereof for use in delaying the onset, improving, or lessening the decline of a symptom of ALS.

In an embodiment, compositions for use according to the invention act by decreasing the rate of neurological deterioration in the subject.

In an embodiment, the subject is administered pridopidine or pharmaceutically acceptable salt thereof and a therapeutically effective amount of Au8 nanocrystalline gold. In an embodiment of the invention, the pridopidine or pharmaceutically acceptable salt thereof and the second compound (CNM-Au8 nanocrystalline gold) are administered in one unit. In another embodiment the pridopidine and the second compound (CNM-Au8 nanocrystalline gold) are administered in more than one unit.

In an embodiment, the amount of pridopidine or pharmaceutically acceptable salt thereof and the amount of the second compound (CNM-Au8 nanocrystalline gold) are administered simultaneously. In an embodiment, the amount of pridopidine or pharmaceutically acceptable salt thereof and the amount of the second compound (CNM-Au8 nanocrystalline gold) are administered contemporaneously.

In another embodiment, the administration of the second compound (CNM-Au8 nanocrystalline gold) precedes the administration of pridopidine or pharmaceutically acceptable salt thereof. In another embodiment, the administration of pridopidine or pharmaceutically acceptable salt thereof precedes the administration of the second compound (CNM-Au8 nanocrystalline gold).

In an embodiment, the subject is receiving CNM-Au8 nanocrystalline gold therapy prior to initiating pridopidine therapy.

In another embodiment, the subject is receiving CNM-Au8 nanocrystalline gold therapy for at least 24 weeks, 28 weeks, 48 weeks, or 52 weeks prior to initiating pridopidine therapy.

In an embodiment, the subject is receiving pridopidine therapy prior to initiating CNM-Au8 nanocrystalline gold therapy. In another embodiment, a subject is receiving pridopidine therapy for at least 24 weeks, 28 weeks, 48 weeks, or 52 weeks prior to initiating CNM-Au8 nanocrystalline gold therapy.

In an embodiment CNM-Au8 nanocrystalline gold is administered orally. In another embodiment, CNM-Au8 nanocrystalline gold is administered in a daily dose between 5-50 mg/day. In another embodiment CNM-Au8 nanocrystalline gold is administered in a daily dose of between 5-10 mg/day, 15-20 mg/day, 15-30 mg/day, 20-30 mg/day. In another embodiment, CNM-Au8 nanocrystalline gold is administered once a day, twice a day or more than twice a day.

In an embodiment, each of the amount of the second compound (CNM-Au8 nanocrystalline gold) when taken alone, and the amount of pridopidine or pharmaceutically acceptable salt thereof when taken alone is effective to treat a subject. In another embodiment, either the amount of the second compound when taken alone, the amount of pridopidine or pharmaceutically acceptable salt thereof when taken alone, is less effective to treat the subject.

In an embodiment, pridopidine or pharmaceutically acceptable salt thereof is administered adjunctively to the second compound (CNM-Au8 nanocrystalline gold). In another embodiment, the second compound is administered adjunctively to pridopidine or pharmaceutically acceptable salt thereof.

In an embodiment, a loading dose of an amount different from the intended dose is administered for a period of time at the start of the periodic administration.

In an embodiment, the combination of the pridopidine or pharmaceutically acceptable salt thereof and the second compound (CNM-Au8 nanocrystalline gold) is effective for enhancing BDNF axonal transport in motor neurons in a subject afflicted with ALS.

In an embodiment, the combination of the pridopidine or pharmaceutically acceptable salt thereof and the second compound (CNM-Au8 nanocrystalline gold) is effective for enhancing ERK activation in motor neurons of a subject afflicted with ALS.

In an embodiment, the combination of the pridopidine or pharmaceutically acceptable salt thereof and the second compound (CNM-Au8 nanocrystalline gold) is effective for preserving neuromuscular junction (NMJ) structure in muscle cells of a subject afflicted with ALS.

In an embodiment, the combination of the pridopidine or pharmaceutically acceptable salt thereof and the second compound (CNM-Au8 nanocrystalline gold) is effective for improving muscle contraction function in a subject afflicted with ALS.

In an embodiment, the combination of the pridopidine or pharmaceutically acceptable salt thereof and the second compound (CNM-Au8 nanocrystalline gold) is effective for improving innervation rate of muscle tissue in a subject afflicted with ALS.

In an embodiment, the combination of the pridopidine or pharmaceutically acceptable salt thereof and the second compound (CNM-Au8 nanocrystalline gold) is effective for enhancing motor neuron axonal growth in a subject afflicted with ALS.

In an embodiment, the combination of the pridopidine or pharmaceutically acceptable salt thereof and the second compound (CNM-Au8 nanocrystalline gold) is effective for enhancing muscle cell survival in a subject afflicted with ALS.

In an embodiment, the combination of the pridopidine or pharmaceutically acceptable salt thereof and the second compound (CNM-Au8 nanocrystalline gold) is effective for reducing muscle fiber wasting or reducing progression of muscle fiber wasting in a subject afflicted with ALS.

In an embodiment, the combination of the pridopidine or pharmaceutically acceptable salt thereof and the second compound (CNM-Au8 nanocrystalline gold) is effective for reducing axonal degeneration in a subject afflicted with ALS.

In an embodiment, the combination of the pridopidine or pharmaceutically acceptable salt thereof and the second compound (CNM-Au8 nanocrystalline gold) is effective for preserving NMJ formation in a subject afflicted with ALS.

In an embodiment, the combination of the pridopidine or pharmaceutically acceptable salt thereof and the second compound (CNM-Au8 nanocrystalline gold) is effective for preserving NMJ structure and function in a subject afflicted with ALS.

In an embodiment, the combination of the pridopidine or pharmaceutically acceptable salt thereof and the second compound (CNM-Au8 nanocrystalline gold) is effective for reducing protein aggregation in a subject afflicted with ALS.

In an embodiment, the combination of the pridopidine or pharmaceutically acceptable salt thereof and the second compound (CNM-Au8 nanocrystalline gold) is effective for attenuating pseudobulbar disease progression, e.g., delaying onset of pseudobulbar impairment, slowing progression of pseudobulbar impairment or improving pseudobulbar function, in a subject afflicted with ALS.

All combinations, sub-combinations, and permutations of the various elements uses described herein are envisaged in the disclosure; the invention is as defined in the claims.

### Pharmaceutically Acceptable Salts

As used herein, "pridopidine" means pridopidine base or a pharmaceutically acceptable salt thereof as well as derivatives, for example deuterium-enriched version of pridopidine and salts. Examples of deuterium-enriched pridopidine and salts and their methods of preparation may be found in U.S. Application Publication Nos. 2013-0197031, 2016-0166559 and 2016-0095847. In certain embodiments, pridopidine is a pharmaceutically acceptable salt, such as the HCl salt or tartrate salt. Preferably, in any embodiments of the invention as described herein, the pridopidine is in the form of its hydrochloride salt.

Examples of pharmaceutically acceptable addition salts include, without limitation, the nontoxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methane sulphonate, the naphthalene-2-sulphonate, the phthalate, the salicylate, the sorbate, the stearate, the succinate, the tartrate, the toluene-p-sulphonate, and the like. Such salts may be formed by procedures well known and described in the art.

"Deuterium-enriched" means that the abundance of deuterium at any relevant site of the compound is more than the abundance of deuterium naturally occurring at that site in an amount of the compound. The naturally occurring distribution of deuterium is about 0.0156%. Thus, in

a "deuterium-enriched" compound, the abundance of deuterium at any of its relevant sites is more than 0.0156% and can range from more than 0.0156% to 100%. Deuterium-enriched compounds may be obtained by exchanging hydrogen with deuterium or synthesizing the compound with deuterium-enriched starting materials.

### Pridopidine Analogs

In some embodiments the uses of this invention make use of a pharmaceutical composition comprising pridopidine or a pharmaceutically acceptable salt thereof in combination with CNM-Au8 nanocrystalline gold. In one embodiment, the composition comprises pridopidine or pharmaceutically acceptable salt thereof and at least one pridopidine analog or pharmaceutically acceptable salt thereof. In another embodiment, the pridopidine analogs are represented by the following structures of compounds 1-7: 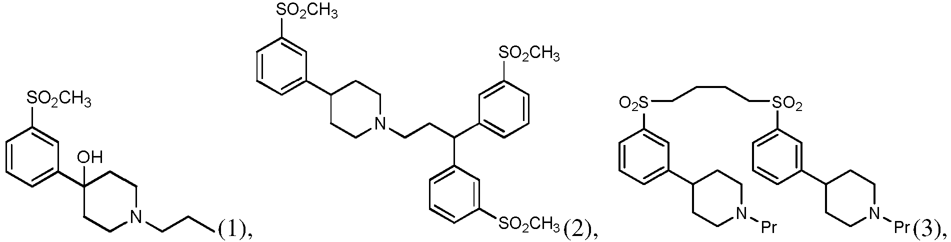 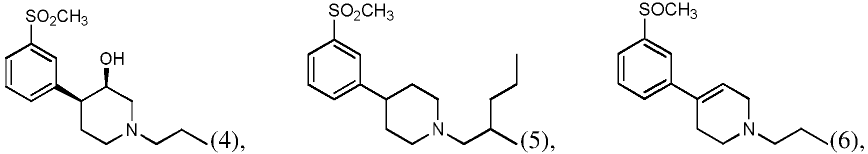 or

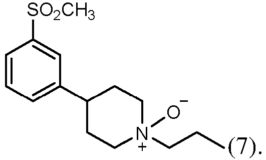

In other embodiments this invention provides for the use of a pharmaceutical composition comprising pridopidine or pharmaceutically acceptable salt thereof and compound 1 or pharmaceutically acceptable salt thereof; or the use of a pharmaceutical composition comprising pridopidine or pharmaceutically acceptable salt thereof and compound 2 or pharmaceutically acceptable salt thereof; or the use of a pharmaceutical composition comprising pridopidine or pharmaceutically acceptable salt thereof and compound 3 or pharmaceutically acceptable salt thereof; or the use of a pharmaceutical composition comprising pridopidine or pharmaceutically acceptable salt thereof and compound 4 or pharmaceutically acceptable salt thereof; or the use of a pharmaceutical composition comprising pridopidine or pharmaceutically acceptable salt thereof and compound 5 or pharmaceutically acceptable salt thereof; or the use of a pharmaceutical composition comprising pridopidine or pharmaceutically acceptable salt thereof and compound 6 or pharmaceutically acceptable salt thereof; or the use of a pharmaceutical composition comprising pridopidine or pharmaceutically acceptable salt thereof and compound 7 or pharmaceutically acceptable salt thereof; or the use of a pharmaceutical composition comprising pridopidine or pharmaceutically acceptable salt thereof and compound 1 and compound 4 or pharmaceutically acceptable salt thereof, in each case in combination with CNM-Au8 nanocrystalline gold. In other embodiments, the concentration of compounds 1, 2, 3, 4, 5, 6 or 7 or pharmaceutically acceptable salt thereof within the composition is between 0.001% w/w to 10% w/w. In other embodiments, the concentration of compounds 1, 2, 3, 4, 5, 6 or 7 or pharmaceutically acceptable salt thereof within the composition is between 0.001% w/w to 0.05% w/w. In other embodiments, the concentration of compounds 1, 2, 3, 4, 5, 6 or 7 or pharmaceutically acceptable salt thereof within the composition is between 0.001% w/w to 0.5% w/w. In other embodiments, the concentration of compounds 1, 2, 3, 4, 5, 6 or 7 or pharmaceutically acceptable salt thereof within the composition is between 0.001% w/w to 0.15% w/w. In other embodiments, the concentration of compounds 1, 2, 3, 4, 5, 6 or 7 or pharmaceutically acceptable salt thereof within the composition is between 0.01% w/w to 0.15% w/w. In other embodiments, the concentration of compounds 1, 2, 3, 4, 5, 6 or 7 or pharmaceutically acceptable salt thereof within the composition is between 0.01% w/w to 0. 5% w/w. In other embodiments, the concentration of compounds 1, 2, 3, 4, 5, 6 or 7 or pharmaceutically acceptable salt thereof within the composition is between 0.01% w/w to 1% w/w. In an embodiment, the pridopidine analog salt is selected from the group consisting of hydrochloride, hydrobromide, nitrate, perchlorate, phosphate, sulphate, formate, acetate, aconate, ascorbate, benzenesulphonate, benzoate, cinnamate, citrate, the embonate, enantate, fumarate, glutamate, glycolate, lactate, maleate, malonate, mandelate, methane sulphonate, naphthalene-2-sulphonate, phthalate, salicylate, sorbate, stearate, succinate, tartrate and toluene-p-sulphonate:

### Pharmaceutical Compositions

While pridopidine for use according to the invention may be administered in the form of the raw compound, preferred administration of pridopidine, optionally in the form of a physiologically acceptable salt, is in a pharmaceutical composition together with one or more adjuvants, excipients, carriers, buffers, diluents, and/or other customary pharmaceutical auxiliaries.

This invention provides the use of pharmaceutical compositions comprising the pridopidine or pharmaceutically acceptable salts or derivatives thereof, together with one or more pharmaceutically acceptable carriers therefore, and CNM-Au8 nanocrystalline gold.

The carrier(s) must be "acceptable" in the sense of being compatible with the other ingredients of the formulation and suitable for administration to a human subject.

As used herein, and unless stated otherwise, each of the following terms shall have the definition set forth below.

CNM-Au8 nanocrystalline gold are small nanocrystals that provide energetic assistance by supporting bioenergetic reactions and eliminating harmful bioproducts of cell metabolism. CNM-Au8 shows neuroprotective effects in preclinical models. CNM-Au8 consists solely of gold nanoparticles, composed of clean-surfaced, faceted, geometrical crystals held in suspension in sodium bicarbonate buffered, pharmaceutical grade water.

As used herein, an "amount" or "dose" of pridopidine as measured in milligrams refers to the milligrams of underivatized pridopidine base present in a preparation, regardless of the form of the preparation. A "dose of 45 mg pridopidine" means the amount of pridopidine in a preparation is sufficient to provide 45 mg of underivatized pridopidine base having a naturally occurring isotope distribution, regardless of the form of the preparation. Thus, when in the form of a salt, e.g. a pridopidine hydrochloride, the mass of the salt form necessary to provide a dose of 45 mg underivatized pridopidine base would be greater than 45 mg due to the presence of the additional salt ion. Similarly, when in the form of a deuterium-enriched derivative, the mass of the derivatized form necessary to provide a dose of 45 mg underivatized pridopidine base having a naturally occurring isotope distribution would be greater than 45 mg due to the presence of the additional deuterium.

By any range disclosed herein, it is meant that all hundredth, tenth and integer unit amounts within the range are specifically disclosed as part of the invention. Thus, for example, 0.01 mg to 50 mg means that 0.02, 0.03 ... 0.09; 0.1; 0.2 ... 0.9; and 1, 2 ... 49 mg unit amounts are included as embodiments of this invention. By any range of time disclosed herein (i.e. weeks, months, or years), it is meant that all lengths of time of days and/or weeks within the range are specifically disclosed as part of the invention. Thus, for example, 3-6 months means that 3 months and 1 day, 3 months and 1 week, and 4 months are included as embodiments of the invention.

As used herein, "about" in the context of a numerical value or range means ±10% of the numerical value or range recited or claimed.

As used herein, "monotherapy" means treatment with a single active agent, for example treatment with pridopidine alone.

As used herein, "adjunctively" means treatment with or administration of an additional compound, with a primary compound, for example for increasing the efficacy or safety of the primary compound or for facilitating its activity.

As used herein, "periodic administration" means repeated/recurrent administration separated by a period of time. The period of time between administrations is preferably consistent from time to time. Periodic administration can include administration, e.g., once daily, twice daily, three times daily, four times daily, weekly, twice weekly, three times weekly, four times a week and so on, etc.

As used herein, "combination" means an assemblage of reagents for use in therapy either by simultaneous or contemporaneous administration. Simultaneous administration refers to administration of an admixture (whether a true mixture, a suspension, an emulsion, or other physical combination) of the pridopidine and a second compound (CNM-Au8 nanocrystalline gold). In this case, the combination may be the admixture or separate containers of the pridopidine and the second compound (CNM-Au8 nanocrystalline gold) that are combined just prior to administration. Contemporaneous administration, or concomitant administration refer to the separate administration of the pridopidine and the second compound (CNM-Au8 nanocrystalline gold) at the same time, or at times sufficiently close together that an additive or preferably synergistic activity relative to the activity of either the pridopidine or the second compound alone is observed or in close enough temporal proximately to allow the individual therapeutic effects of each agent to overlap.

As used herein, "add-on" or "add-on therapy" means an assemblage of reagents for use in therapy, wherein the subject receiving the therapy begins a first treatment regimen of one or more reagents prior to beginning a second treatment regimen of one or more different reagents in addition to the first treatment regimen, so that not all of the reagents used in the therapy are started at the same time.

As used herein, "effective" when referring to an amount of pridopidine refers to the quantity of pridopidine that is sufficient to yield a desired therapeutic response. In a preferred embodiment, the quantity of pridopidine administered does not result in adverse side-effects (such as toxicity, irritation, or allergic response).

"Administering to the subject" or "administering to the (human) patient" means the giving of, dispensing of, or application of medicines, drugs, or remedies to a subject/patient to relieve, cure, or reduce the symptoms associated with a disease, disorder, or condition, e.g., a pathological condition.

"Treating" as used herein encompasses inducing inhibition, regression, or stasis of a disease or disorder, or lessening, suppressing, inhibiting, reducing the severity of, eliminating, or substantially eliminating, or ameliorating a symptom of the disease or disorder.

"Inhibition" of disease progression or disease complication in a subject means preventing or reducing the disease progression and/or disease complication in the subject.

A "symptom" associated with a disease or disorder includes any clinical or laboratory manifestation associated with the disease or disorder and is not limited to what the subject can feel or observe.

As used herein, "a subject afflicted with" a disease, disorder or condition means a subject who has been clinically diagnosed to have the disease, disorder, or condition.

Glial cell-derived neurotrophic factor (GDNF) is a protein encoded by the GDNF gene and is believed to promote the survival of many types of neurons them.

Brain-derived neurotrophic factor (BDNF) is a protein produced by neurons and serves to keep functioning and to promote the growth of neurons and neurogenesis.

Throughout this application, certain publications and patent application publications are referenced. Full citations for the publications may be found immediately preceding the claims.

This invention will be better understood by reference to the Experimental Details which follow, but those skilled in the art will readily appreciate that the specific experiments detailed are only illustrative of the invention as defined in the claims which follow thereafter.

### EXPERIMENTAL DETAILS

### Experiment 1: The effect of pridopidine in axonal transport assays.

Healthy motor neurons (MN) extend axons over long distances and through varying extracellular microenvironments to form synapses with muscles. The ability of the neuron to maintain this specialized morphology depends on cytoskeletal elements and continuous transport of proteins and organelles to and from the cell body. Cytoskeletal alterations are a major pathway implicated in the pathogenesis of ALS affecting axonal transport, growth, and neuromuscular junction (NMJ) function (Eykens and Robberecht, 2015). Alterations in axonal transport are one of the first cellular processes that occur in neurodegenerative disease, including ALS.

Axonal transport was evaluated using an in vitro compartmentalized system of microfluidic chambers (MFC) that separates neuronal cell bodies from their axons and synapses, enabling the study of retrograde/anterograde transport of fluorescently-labelled molecules (e.g. Qdot-BDNF) by specific monitoring and manipulation of cellular microenvironments (Figure 1A; Zahavi 2015; Ionescu 2016).

Quantum-Dot labeled BDNF (Qdot BDNF) is retrogradely transported in axons of motor neurons grown from spinal cord explants in a microfluidic chamber (MFC). A MFC was used to analyze Qdot BDNF axonal transport (Figure 1A). Axonal transport of BDNF in the SOD1 model (SOD1G93A) for ALS is disrupted (Bilsland 2010; Perlson 2009; De Vos 2007). The effect of pridopidine on transport of Qdot BDNF along the axons of motor neurons was assessed in spinal cord explants from embryonic day E12.5 SOD1 G93A and WT littermate mice (WT). Experimental workflow for the axonal transport assay (from left to right, Figure 1B): SOD1G93A or wild Type (WT) spinal cord explants were plated in the MFC. At about 5 days post plating, axons begin to cross over into the distal compartment. On day 6 post plating, an amount of pridopidine is added to both compartments. On day7, Qdot-BDNF is added to the distal compartment and axonal transport imaged using a high-resolution spinning-disk confocal microscope. Schematic illustration of microfluidic chamber system (Figure 1A): Explants planted in the proximal compartment extend axons to the distal compartment, where Qdot-BDNF is applied exclusively prior to visualization.

Spinning disk confocal microscopy was used to track Qdot BDNF along the axons of motor neuron explant cultures. Time lapse images of Qdot-BDNF axonal transport as acquired at 60X magnification (Figure 1C). Arrowheads point to a single Qdot-BDNF particle that is retrogradely transported (left) towards the cell body. Scale bar: 10µm. Bottom panel shows a kymograph of a complete Qdot-BDNF time-lapse movie that plots movement along the axon (x axis) as a function of time (y axis). Scale bars: horizontal 10µm; vertical 100 seconds (Figure 1C)

Vehicle and pridopidine were added to both compartments at 2 concentrations (0.1 µM, and 1µM) on experimental day 6, and Qdot BDNF was added to the distal compartment after overnight incubation with pridopidine (Figures 1A and 1B). Six independent biological repeats, from 6 different cultures were tested so that from each culture and explant with neurons/glia ~250 BDNF particles were followed along the axons in the grooves. Velocity refers to the movement of a single BDNF particle. The experiment was repeated with MNs from mice in which the sigma 1 receptor (S1R) was genetically deleted (S1R KO or S1R -/-) (Langa, 2003). Ventral spinal cord sections from S1R-/- mice embryos were cultured and plated in the MFC as described above, and the axonal transport of Qdot-BDNF was analyzed.

SOD1G93 and S1R-/- explants with or without pridopidine were compared to wild-type littermate controls (WT).

Qdot-BDNF particle tracking was performed on Bitplane Imaris, using the semi-automated spot tracking function. Inclusion criteria for particle analysis: track duration >10 frames; average velocity ≥ 0.2µm/sec; stop duration: speed < 0.1µm/sec for 3 frames. Data were then exported to MATLAB for further analysis of particle transport including Instantaneous Velocities (Figure 2A) from 6 independent cultures; and Stop count (Figure 2B).

### Results:

Figure 2A demonstrates that pridopidine enhances BDNF axonal transport instantaneous velocity in SOD1G93A motor neurons. Instantaneous velocity of BDNF retrograde transport is reduced in SOD1G93A motor neurons. SOD1G93A MNs show slower velocities vs the WT MNs. Pridopidine treatment accelerates the instantaneous velocities in SOD1G93A MNs (0.1µM and 1µM). Application of 25µM or 100µM Riluzole, the standard of care for ALS patients, to SOD1G93A MNs does not affect the instantaneous velocities. S1R-/- MNs demonstrate reduced velocity of BDNF axonal transport. Pridopidine at either 0.1 µM or 1µM is not able to recover these defects in S1R KO MNs indicating the effect of pridopidine is mediated by the S1R (Figure 2A).

Particle stop count (number of counted stops of Qdot-BDNF per second) is increased in SOD1G93A MNs compared to WT MNs. Pridopidine reduces the number of pauses during axonal transport in SOD1G93A MNs (both 0.1µM and 1µM). Pridopidine is unable to rescue particle stop count p of Qdot-BDNF in SIR-/- MNs, indicating that pridopidine's effect is mediated by the S1R (Figure 2B). Data are shown as mean ± SEM. ** p-value < 0.01, *** p-value < 0.001, **** p-value < 0.0001. (Student's t-test).

These results demonstrate that pridopidine enhances BDNF axonal transport in SOD1G93A motor neurons and corrects ALS related deficits.

### Experiment 2: The effect of pridopidine on axon-muscle growth/degeneration assays.

An early event in the pathogenesis of ALS is axonal degeneration. The compartmental co-culture microfluidic chamber system was used to determine whether pridopidine alters axonal degeneration (Figure 3). Primary muscle cells from pre-symptomatic (P60) WT or SOD1G93A mice were cultured. On day 6 primary skeletal myoblasts were cultured in the distal compartment of a MFC. About six days later (day 12), ventral spinal cord explants from WT or SOD1G93A E12.5 mouse embryos that express HB9-GFP (a specific motor neuron marker) were plated in the proximal compartment, followed by application of pridopidine or vehicle to both compartments. Pridopidine was refreshed every other day. Two days post explant plating (day 14), motor axon growth and degeneration were evaluated using live imaging on a spinning disc confocal system. Axonal growth was tracked by imaging every 10 min for 8 hrs. Experiments were repeated three times.

### Results:

The data demonstrate that pridopidine increases axonal growth (Figure 4). Myocytes carrying the SOD1G93A mutation have a reduced number of healthy axons that are able to cross into the distal compartment of the microfluidic compartmental chamber as compared with WT myocytes. Treatment with 1 µM pridopidine (furthest right bar) significantly increase the number of SOD1G93A axons crossing into the distal compartment (compartment with muscle cells). (Y axis is average number of grooves with axons crossing into muscle compartment). Data are shown as mean ± SEM. * p-value < 0.05; (Student's t-test).

These results demonstrate that pridopidine enhances axonal growth in ALS neurons.

### Experiment 3: Assessment of the effect of pridopidine on Neuromuscular junction (NMJ) formation and function.

Synapses are the earliest cellular compartment disrupted in ALS. To test the ability of pridopidine to affect synapse function in an ALS model, cultures from Experiment 2 described above were grown for approximately four additional days (day 18) until axons extend into the distal compartment and generate NMJs. In this co-culture, MN axons formed NMJs on fully differentiated primary myocytes. These can be observed by the co-localization of the post-synaptic marker located in the muscle (AchR, acetyl choline receptor) with the Hb9:GFP neuronal marker. Figure 5A: Upper panel: Phase-contrast microscope image of a myocyte in the distal compartment connected by axons (arrowheads). Scale bar: 20µm. Lower panel: High magnification images of myocyte:MN contact points reveal the formation of NMJs as seen by co-localization of post synaptic AChR with HB9:GFP axons and 3-dimensional co-localization of pre and post-synaptic markers (coloc).

To evaluate NMJ function, movies of muscle contraction were acquired at a frame rate of 30 frames per second for 1000 frames (Figure 5B). Muscle contraction traces as extracted from intensity over time measurements of muscle contraction show the flat trace of a non-contracting, immobile myocyte (upper), and the trace of a contracting myocyte demonstrating multiple bursting events (lower).

To study the effect of pridopidine on MN and NMJ formation and function, either 0.1 or 1 µM pridopidine or vehicle were added. Measurement of % innervation and innervation-induced contraction in myotubes was evaluated using live cell imaging as reported before (Ionescu 2015; Zahavi 2015). Briefly, contractile activity of muscles in the distal compartment of the MFC, which were overlapped by at least one axon was examined. Muscles were categorized into two groups: 'Contracting' or 'Non-contracting', depending on their motile activity during the movie. The motility of muscles was validated by generating intensity-over-time plots for each muscle (Figure 5b). The number of contracting muscle fibers per chamber was divided by the total number of muscle fibers analyzed in the same chamber, yielding the percentage of contracting myotubes as an output for NMJ activity.

### Results:

Pridopidine enhances muscle innervation and increases NMJ function as measured by an increase in the % of contracting myocytes. Innervation rate of muscles carrying the SOD1 mutation is lower compared to WT (wild type) muscles (20% innervation compared to ~ 40% in WTs). Pridopidine 1 µM significantly increases innervation rate of muscles carrying SOD1 mutation to near WT levels (Figure 6).

The percent of contracting myotubes is decreased in SOD1 myocytes innervated with WT MNs compared to WT myocytes innervated with WT MNs. Treatment of SOD1G93A myocytes co-cultured with pridopidine (0.1µM and 1µM) significantly increases the percentage of contracting myocytes and restores neuromuscular activity to WT levels. Both WT and SOD1 myocytes demonstrate reduced contractility when innervated with S1R-/- MNs. Combination of S1R-/- MNs with WT myocytes results in a low number of contracting myotubes compared to WT MNs. Application of 0.1µM pridopidine to S1R-/- co-cultures does not restore the neuromuscular activity, as seen for the same concentration of pridopidine in co-cultures with WT neurons, indicating the effect of pridopidine is mediated via the S1R. Data are shown as mean ± SEM. * p-value < 0.05; ** p-value < 0.01, *** p-value < 0.001, **** p-value < 0.0001. (Student's t-test).

### Experiment 4: Pridopidine effect on activation of ERK in WT and SOD1G93A MNs

The ERK pathway promotes numerous cellular functions including proliferation and differentiation. ERK phosphorylation (activation) in neurons is associated with neurotrophic signaling, such as BDNF, which promotes neuroprotection and neuronal survival (Bonni 1999). It was previously established that pridopidine enhances BDNF signaling in rat striatum through the S1R, which in turn, enhances ERK activation (Geva 2016). Primary MN cultures at 2DIV were starved overnight in neurotrophin- and serum-free medium (PNB). The following day, cultures were treated for 30 minutes with pridopidine or with BDNF as a positive control and the levels of ERK and phosphorylated ERK proteins were measured.

### Results:

Pridopidine induces a significant increase in phosphorylated ERK (pERK) (0.1µM and 1 µM), as early as 30 minutes after application in WT (left panel) and SOD1G93A (middle panel) MN cultures. Pridopidine had no effect in S1R-/- MN cultures (right panel) indicating pridopidine's activation of ERK is mediated via the S1R (Figure 8A). Quantification of pERK reveals ~ 3.5 and ~4- fold increase in WT MNs following 0.1µM and 1µM pridopidine, respectively. SOD1G93A exhibits ~2.9 and ~8.5-fold increase in pERK following 0.1µM and 1µM pridopidine, respectively. Data are shown as the mean pERK/ERK ratios ± SEM. * p-value < 0.05, ~ p-value<0.1 (Student's t-test) (Figure 8B).

### Experiment 5: Effect of pridopidine on mutant SOD1 aggregates in the spinal cord of SOD1G93A mice.

Pridopidine induces neuroprotective properties by activation of the S1R, as demonstrated for its effect on axonal transport, axonal degeneration, NMJ function and ERK activation. The S1R resides on the ER membrane in close proximity to the mitochondrial outer membrane, where the mutant SOD1 protein tends to aggregate in the spinal cord of SOD1G93A mice (Millecamps and Julien 2013).

Pre-symptomatic SOD1G93A mice (5 weeks of age) and WT controls were treated with either saline or 30 mg/kg pridopidine, by daily s.c. (subcutaneous) administration for 11 weeks (until 16 weeks of age). At the end of the experiment, lumbar spinal cords (L1-L6) were extracted, fixed, and embedded for cryosectioning. Next, 10µM sections were prepared and stained with NSC500 dye to visualize SOD1 aggregates (Hammarström 2010). The in vivo effect of pridopidine treatment on the number of mutant SOD1 aggregates in grey (GM) and white matter (WM) of spinal cord was evaluated.

### Results:

Figure 9A- Left panel: low magnification representative images of fluorescently labeled spinal cords for 3 mouse groups. Right panel: high magnification images for the regions marked in the left panel by a square. Scale bars: Left panel: 500µm; Right panel 50µm. Top to bottom: WT vehicle, SOD1G93A vehicle, SOD1G93A 30 mg/kg, all stained with NSC500 dye to label mutant SOD1 protein aggregates.

A significant increase in the number of mSOD1 aggregates is observed in both the gray and white matter of the spinal cords of SOD1G93A mice compared with WT mice. Pridopidine at 30 mg/kg significantly reduces the number of aggregates in both the gray (Figure 9B) and white matter (Figure 9C) of SOD1G93A spinal cords by ~50% (Figures 9A-9C). Data are shown as the mean ± SEM. * p-value < 0.05; ** p-value < 0.01 (one-way ANOVA followed by Fisher's LSD post hoc tests). (Figures 9B-9C y-axis is number of NSC500-positive SOD1 aggregates per squared mm).

### Experiment 6: Pridopidine effect of muscle fiber atrophy and NMJ preservation in-vivo.

NMJ disruption and the subsequent skeletal muscle wasting are two main pathologies of ALS. The effect of pridopidine on muscle fiber atrophy and preservation of NMJs was evaluated in-vivo. Pre-symptomatic SOD1G93A mice and WT controls (5 weeks old) were treated with either saline or pridopidine 30 mg/kg, by daily s.c administration for 11 weeks. The Gastrocnemius muscles from vehicle or pridopidine-treated (30 mg/kg s.c.) mice were extracted from the SOD1G93A and WT mice at age 16 weeks. Muscle cross-sections were stained with Hematoxylin & Eosin (H& E), and the mean muscle fiber diameter was quantified for each group (Figure 10a). NMJ preservation was evaluated by confocal imaging of co-localizing pre (neuronal, NFH+Synapsin-I - and post-synaptic (muscular, AchR (BTX) markers and counting the number of fully innervated NMJs in gastrocnemius muscles (Figure 11A).

### Results:

Figure 10A: Representative images of H&E-stained cross-sections from Gastrocnemius muscle of mice from 3 groups: WT-vehicle treated, SOD1G93A-vehicle treated, and SOD1G93A-30 mg/kg pridopidine treated mice. Muscle histology of SOD1G93A-vehicle mice is poor and reveals a smaller diameter of muscle fiber as compared with WT-vehicle (Figures10A-10B). Pridopidine (30 mg/kg, s.c daily administration) leads to a significant ~ 4µm increase in the muscle fiber diameter in SOD1G93A (Figure 10B).

Muscles of SOD1G93A vehicle-treated mice demonstrate the expected massive~60% loss of NMJ and morphological changes in the post-synaptic apparatus compared to WT mice (Figures 11A-11B). Strikingly, Pridopidine treatment limits the loss of NMJs in SOD1G93A mice to ~20%. Data are shown as mean ± SEM. * p-value < 0.05; ** p-value < 0.01; *** p-value < 0.001 (double-blind Student's t test).

Overall, these results demonstrate that pridopidine exerts neuroprotective effects in cellular and animal models of ALS.

In-vitro, in SOD1G93A MNs, pridopidine enhances BDNF axonal transport, upregulates ERK activation, enhances axonal growth, restores muscle innervation, and improves NMJ formation and function. These neuroprotective effects are mediated by the S1R as a genetic deletion of the S1R gene abolished pridopidine's effects. In-vivo pridopidine treatment of SOD1G93A ALS mice reduces mutant SOD1 aggregation in the spinal cord (one of the hallmark disease phenotype), increases the ALS-reduced muscle fiber diameter and preserves the degenerated NMJs observed in diseased tissue. These data support the use of pridopidine as neuroprotective agent and the S1R as a therapeutic target for the treatment of ALS patients.

In the figures, abbreviations are as follows: Geno. = genotype (i.e. wild type (WT), mutant SOD1), Prido. = pridopidine, mpk = milligram per kilogram.

### Experiment 7: Effect of pridopidine on neurite length and neuronal survival in C9orf72 iPSC-derived motor neurons.

The effect of pridopidine alone on neurite length and neuron survival is evaluated in a unique human transcription-factor induced pluripotent stem cells (iPSCs) differentiation line. iPSCs with the ALS mutation C9orf72 and isogenic controls are differentiated into motor neurons by doxycycline-induced activation of an integrated transcription factor cassette (Ngn2, Isl1, and Lhx3). Neuronal survival and neurite length are assessed by high-content image analysis using the Incucyte system. C9orf72 cells demonstrate reduced survival and decreased axonal growth compared to healthy isogenic control cells.

The effect of pridopidine is assessed in C9orf72 iPSC-derived MNs and isogenic controls using the Incucyte system.

### Experiment 8: Effect of pridopidine in combination with another drug on C9ORF72 human iPSC-derived motor neuron survival and neurite length.

The effect of pridopidine in combination with an additional drug is evaluated on neuronal survival and neurite length of C9orf72 iPSC-derived MNs using high-content analysis in the Incucyte system. The drug evaluated will be CNM-Au8 nanocrystalline gold.

The combination of pridopidine and the additional drug demonstrates either an additive or a synergistic effect on neurite outgrowth and cell survival.

### Experiment 9. Treatment of ALS in a human subject with combination of pridopidine and another drug.

Periodically orally administering of pridopidine together with an additional drug provides a clinically meaningful advantage in reducing the symptoms of ALS in human subjects afflicted with ALS. Pridopidine combination therapy provides efficacy in treating the patient without undue adverse side effects and is effective in at least one of the following embodiments
1. The therapy is effective in improving a symptom of ALS;
2. The therapy is effective in enhancing cell survival and neurite growth.
3. The therapy is effective in enhancing BDNF axonal transport in motor neurons and/or enhancing ERK activation.
4. The therapy is effective in improving NMJ formation and preservation, preserving NMJ structure, preserving NMJ function and/or improving innervation rate of muscle tissue;
5. The therapy is effective in enhancing motor neuron axonal growth and/or reducing axonal degeneration, including motor neuron axonal degeneration;
6. The therapy is effective in enhancing muscle cell survival, enhancing muscle fiber diameter and function, reduce progression of muscle fiber wasting, and/or improve muscle contraction; and or
7. The therapy is effective in reducing SOD1 aggregation and/or lessening pseudobulbar disease progression.

In some patients, the attending physician administers pridopidine and a second compound, wherein the second compound is CNM-Au8 nanocrystalline gold.

### REFERENCES

Bilsland et al. (2010) Deficits in axonal transport precede ALS symptoms in vivo. Proc Natl Acad Sci U S A. 107(47):20523-8.
Bonni A, et al. (1999) Cell survival promoted by the Ras-MAPK signaling pathway by transcription-dependent and -independent mechanisms. Science 286:1358-1362.
Bozzoni et al. (2016) Amyotrophic lateral sclerosis and environmental factors", Funct. Neurol. 31(1):7-19.
Chiò, A. et al. (2009) Prognostic Factors in ALS: A Critical Review. Amyotrophic Lateral Sclerosis 10(5-6): 310-323.
Chiò, A., et al. (2013) Global Epidemiology of Amyotrophic Lateral Sclerosis: A Systematic Review of the Published Literature. Neuroepidemiology 2013;41:118-130.
De Vos et al. (2007) Familial amyotrophic lateral sclerosis-linked SOD1 mutants perturb fast axonal transport to reduce axonal mitochondria content. Hum Mol Genet. 16(22):2720-2728.
Eykens C, Robberecht W (2015) The genetic basis of amyotrophic lateral sclerosis: recent breakthroughs. Adv Genomics Genetics 5:327-345.
Geva et al. (2016). Pridopidine activates neuroprotective pathways impaired in Huntington Disease. HMG 25(18): 3975-87.
Gordon, Paul H., et al. (2011) The Range and Clinical Impact of Cognitive Impairment in French Patients with ALS: A Cross-Sectional Study of Neuropsychological Test Performance. Amyotrophic Lateral Sclerosis 12: 372-378.
Guidance for Industry. In vivo drug metabolism/drug interaction studies - study design, data analysis, and recommendations for dosing and labeling, U.S. Dept. Health and Human Svcs., FDA, Ctr. for Drug Eval. and Res., Ctr. For Biologics Eval. and Res., Clin. Pharm., Nov. 1999 <http://www.fda.gov/cber/gdlns/metabol.pdf>.
Gundlach, A. L.,et al. (1986) Autoradiographic Localization of Sigma Receptor Binding Sites in Guinea Pig and Rat Central Nervous System with (+)3H-3-(3-Hydroxyphenyl)-N-(1-Propyl)Piperidine. Journal of Neuroscience 6(6): 1757-1770.
Hammarström P, et al, (2010) A fluorescent pentameric thiophene derivative detects in vitro-formed prefibrillar protein aggregates. Biochemistry 49:6838-45
Ionescu et al. (2016) Compartmental microfluidic system for studying muscle-neuron communication and neuromuscular junction maintenance. (2016) European Journal of Cell Biology 95(2) 69-88.
Ionescu et al. (2019) Targeting the Sigma-1 Receptor via Pridopidine Ameliorates Central Features of ALS Pathology in a SOD1G93A Model. Cell Death and Disease 10:210.
Jaiswal, Manoj Kumar. (2019) Riluzole and Edaravone: A Tale of Two Amyotrophic Lateral Sclerosis Drugs. Medicinal Research Reviews 1-16.
Kourrich, S. et al. (2012) The Sigma-1 Receptor: Roles in Neuronal Plasticity and Disease. Trends in Neurosciences 35 (12): 762-71.
Langa F et al. (2003) Generation and phenotypic analysis of sigma receptor type I (sigma 1) knockout mice. Eur J Neurosci. 18:2188-96.
Lomen-Hoerth, C., et al. (2003) Are Amyotrophic Lateral Sclerosis Patients Cognitively Normal? Neurology 60:1094-1097.
Martel et al. (2016) From animal models to human disease: a genetic approach for personalized medicine in ALS, Acta Neuropathol. Commun. 4(1):70.
Mavlyutov, T. A., et al. (2010) The Sigma-1 Receptor Is Enriched in Postsynaptic Sites of C-Terminals in Mouse Motoneurons. An Anatomical and Behavioral Study. Neuroscience 167 (2): 247-55.
Mavlyutov, T. A., et al. (2012) Development of the Sigma-1 Receptor in C-Terminals of Motoneurons and Colocalization with the N,N'-Dimethyltryptamine Forming Enzyme, Indole-N-Methyl Transferase. Neuroscience 206: 60-68.
Millecamps and Julien (2013) Axonal transport deficits and neurodegenerative diseases. Nat Rev Neurosci. 14:161-76.
Olney, R. K., et al. (2005) The Effects of Executive and Behavioral Dysfunction on the Course of ALS." Neurology 65:1774-1777.
Perlson, et al. (2009) A Switch in Retrograde Signaling from Survival to Stress in Rapid Onset Neurodegeneration. J Neurosci. 2009 29(31): 9903-9917.
Peters et al. (2015) Emerging mechanisms of molecular pathology in ALS, J. Clin. Invest. 125(5):1767-1779.
Ryskamp, Daniel A., et al. (2019) Neuronal Sigma-1 Receptors: Signaling Functions and Protective Roles in Neurodegenerative Diseases. Frontiers in Neuroscience 13:862.
Ryskamp, et al (2017) The sigma-1 receptor mediates the beneficial effects of pridopidine in a mouse model of Huntington disease. Neurobiol of Disease 97(Pt A):46-59.
Su, Tsung Ping, et al. (2010) The Sigma-1 Receptor Chaperone as an Inter-Organelle Signaling Modulator. Trends in Pharmacological Sciences Vol.31 No.12.
Waterhouse, Rikki N., et al. (1997) Halogenated 4-(Phenoxymethyl)Piperidines as Potential Radiolabeled Probes for σ-1 Receptors: In Vivo Evaluation of [123I]-1-(Iodopropen-2-yl)-4-[(4-Cyanophenoxy)Methyl] Piperidine. Journal of Medicinal Chemistry 40, 1657-1667.
Zahavi, et al. (2015) A compartmentalized microfluidic neuromuscular co-culture system reveals spatial aspects of GDNF functions. J. Cell Sci. 128, 1241-1252.
Zou et al. (2016) Toward precision medicine in amyotrophic lateral sclerosis, Ann. Transl. Med. 4(2):27.
Zou et al. (2017) Genetic epidemiology of amyotrophic lateral sclerosis: a systematic review and meta-analysis. Journal of Neurology, Neurosurgery & Psychiatry, 88(7), 540-549.Riluzole
- Drug Summary, PDR (Prescribers' Digital Reference), www.pdr.net/drug-summary/Rilutek-riluzole-526 accessed July 28, 2017
Edaravone- Drug Summary, PDR (Prescribers' Digital Reference), www.pdr.net/drug-summary/Radicava-edaravone-24080 accessed July 28, 2017
Dextromethorphan hydrobromide/quinidine sulfate - Drug Summary, PDR (Prescribers' Digital Reference), http://www.pdr.net/drug-summary/Nuedexta-dextromethorphan-hydrobromide-quinidine-sulfate-1344.3281 accessed August 14, 2017
U.S. Patent No. 7,923,459
U.S. Patent No. RE46117
PCT Application Publication No. WO 2016/138135
PCT Application Publication No. WO 2017/015609

## Claims

1. A combination of a composition comprising pridopidine or pharmaceutically acceptable salt thereof, and
a second composition comprising CNM-Au8 nanocrystalline gold,
for use in treating amyotrophic lateral sclerosis (ALS) in a subject.

2. The combination for use according to claim 1, wherein the ALS is sporadic or familial ALS.

3. The combination for use according to claim 1, wherein the combination is effective to delay the onset, lessen the decline, or improve a symptom of the ALS in the subject.

4. The combination for use according to claim 3, wherein the symptom is muscle stiffness, muscle weakness, muscle wasting, muscle cramps, difficulty speaking, difficulty swallowing, difficulty breathing, difficulty chewing, difficulty walking, fasciculations, worsening posture, respiratory function, muscle strength, bulbar function, speech, salivation, difficulty swallowing, difficulty in handwriting, difficulty in cutting food and handling utensils, difficulty in dressing, dyspnea, orthopnea.

5. The combination for use according to claim 1, wherein the combination is effective to enhance BDNF axonal transport in motor neurons, enhance ERK activation in motor neurons, improve NMJ formation and preservation, preserve NMJ structure, enhance NMJ function, improve innervation rate of muscle tissue, enhance motor neuron axonal growth, reduce axonal degeneration, reduce motor neuron axonal degeneration, enhance muscle cell survival, enhance muscle fiber diameter and function or reduce SOD1 aggregation.

6. The combination for use according to claim 1, wherein the combination further lessens pseudobulbar disease progression, reduces progression of muscle fiber wasting, and/or improves muscle contraction in the subject afflicted with ALS.

7. The combination for use according to any of claims 1-6, wherein the composition comprising pridopidine or pharmaceutically acceptable salt thereof and the second composition are administered daily, twice daily, twice a week, three times a week or more often than once daily.

8. The combination for use according to any of claims 1-7, wherein the composition comprising pridopidine or pharmaceutically acceptable salt thereof is administered orally or via gastric tube.

9. The combination for use according to any of claims 1-8, wherein the amount of pridopidine or pharmaceutically acceptable salt thereof administered is 10 mg per day to 135 mg per day.

10. The combination for use according to any of claims 1-9, wherein the pridopidine is pridopidine hydrochloride.

11. The combination for use according to any of claims 1-10,
wherein the amount of CNM-Au8 nanocrystalline gold administered is between 5-50 mg/day and is administered orally.

12. The combination for use according to any of claims 1-11, wherein the administration of the second composition precedes the administration of the composition comprising pridopidine or pharmaceutically acceptable salt thereof; or
wherein the administration of the composition comprising pridopidine or pharmaceutically acceptable salt thereof precedes the administration of the second composition; or
wherein the composition comprising pridopidine or pharmaceutically acceptable salt thereof is administered adjunctively to the second composition; or
wherein the second composition is administered adjunctively to the composition comprising pridopidine or pharmaceutically acceptable salt thereof.

## Patentansprüche

1. Kombination von einer Zusammensetzung, umfassend Pridopidin oder ein pharmazeutisch unbedenkliches Salz davon, und
einer zweiten Zusammensetzung, umfassend nanokristallines CNM-Au8-Gold,
zur Verwendung bei der Behandlung von amyotropher Lateralsklerose (ALS) bei einem Probanden.

2. Kombination zur Verwendung nach Anspruch 1, wobei die ALS sporadische oder familiäre ALS ist.

3. Kombination zur Verwendung nach Anspruch 1, wobei die Kombination zur Verzögerung des Einsetzens, Verminderung des Verfalls oder Verbesserung eines Symptoms der ALS bei dem Probanden wirksam ist.

4. Kombination zur Verwendung nach Anspruch 3, wobei es sich bei dem Symptom um Muskelsteifheit, Muskelschwäche, Muskelschwund, Muskelkrämpfe, Sprachbeschwerden, Schluckbeschwerden, Atembeschwerden, Kaubeschwerden, Gehbeschwerden, Faszikulationen, eine sich verschlechternde Körperhaltung, Atemfunktion, Muskelkraft, Bulbusfunktion, Sprache, Speichelsekretion, Schluckbeschwerden, Schwierigkeiten beim Schreiben mit der Hand, Schwierigkeiten beim Schneiden von Lebensmitteln und Handhaben von Besteck, Schwierigkeiten beim Anziehen, Dyspnoe, Orthopnoe handelt.

5. Kombination zur Verwendung nach Anspruch 1, wobei die Kombination zum Steigern des axonalen Transports von BDNF in Motoneuronen, Steigern der ERK-Aktivierung in Motoneuronen, Verbessern der NMJ-Bildung und -Konservierung, Konservieren der NMJ-Struktur, Steigern der NMJ-Funktion, Verbessern der Innervationsrate von Muskelgewebe, Steigern des axonalen Wachstums von Motoneuronen, Verringern der axonalen Degeneration, Verringern der axonalen Degeneration von Motoneuronen, Steigern des Muskelzellüberlebens, Steigern des Muskelfaserdurchmessers und der Muskelfaserfunktion oder Verringern der SODI-Aggregation wirksam ist.

6. Kombination zur Verwendung nach Anspruch 1, wobei die Kombination ferner die Progression einer pseudobulbären Erkrankung mindert, die Progression von Muskelfaserschwund verringert und/oder die Muskelkontraktion bei dem unter ALS leidenden Probanden verbessert.

7. Kombination zur Verwendung nach einem der Ansprüche 1-6, wobei die Zusammensetzung, umfassend Pridopidin oder ein pharmazeutisch unbedenkliches Salz davon, und die zweite Zusammensetzung täglich, zweimal täglich, zweimal wöchentlich, dreimal wöchentlich oder mehr als einmal täglich verabreicht werden.

8. Kombination zur Verwendung nach einem der Ansprüche 1-7, wobei die Zusammensetzung, umfassend Pridopidin oder ein pharmazeutisch unbedenkliches Salz davon, oral oder mittels einer Magensonde verabreicht wird.

9. Kombination zur Verwendung nach einem der Ansprüche 1-8, wobei die verabreichte Menge von Pridopidin oder einem pharmazeutisch unbedenklichen Salz davon 10 mg täglich bis 135 mg täglich beträgt.

10. Kombination zur Verwendung nach einem der Ansprüche 1-9, wobei das Pridopidin Pridopidin-Hydrochlorid ist.

11. Kombination zur Verwendung nach einem der Ansprüche 1-10, wobei die verabreichte Menge von nanokristallinem CNM-Au8-Gold zwischen 5 und 50 mg/Tag liegt und oral verabreicht wird.

12. Kombination zur Verwendung nach einem der Ansprüche 1-11, wobei die Verabreichung der zweiten Zusammensetzung der Verabreichung der Zusammensetzung, umfassend Pridopidin oder ein pharmazeutisch unbedenkliches Salz davon, vorangeht; oder
wobei die Verabreichung der Zusammensetzung, umfassend Pridopidin oder ein pharmazeutisch unbedenkliches Salz davon, der Verabreichung der zweiten Zusammensetzung vorangeht; oder
wobei die Verabreichung der Zusammensetzung, umfassend Pridopidin oder ein pharmazeutisch unbedenkliches Salz davon, begleitend mit der zweiten Zusammensetzung verabreicht wird; oder
wobei die zweite Zusammensetzung begleitend mit der Zusammensetzung, umfassend Pridopidin oder ein pharmazeutisch unbedenkliches Salz davon, verabreicht wird.

## Revendications

1. Combinaison d'une composition comprenant de la pridopidine ou un de ses sels pharmaceutiquement acceptables, et
d'une deuxième composition comprenant de l'or nanocristallin CNM-Au8, pour une utilisation dans le traitement de la sclérose latérale amyotrophique (SLA) chez un sujet.

2. Combinaison destinée à être utilisée selon la revendication 1, dans laquelle la SLA est une SLA sporadique ou familiale.

3. Combinaison destinée à être utilisée selon la revendication 1, dans laquelle la combinaison est efficace pour retarder l'apparition, atténuer le déclin ou améliorer un symptôme de la SLA chez le sujet.

4. Combinaison destinée à être utilisée selon la revendication 3, dans laquelle le symptôme est une raideur musculaire, une faiblesse musculaire, une atrophie musculaire, des crampes musculaires, des difficultés à parler, des difficultés à avaler, des difficultés à respirer, des difficultés à mâcher, des difficultés à marcher, des fasciculations, une détérioration de la posture, de la fonction respiratoire, de la force musculaire, de la fonction bulbaire, de la parole, de la salivation, des difficultés à avaler, des difficultés à écrire, des difficultés à couper les aliments et à manipuler des ustensiles, des difficultés à s'habiller, une dyspnée, une orthopnée.

5. Combinaison destinée à être utilisée selon la revendication 1, dans laquelle la combinaison est efficace pour améliorer le transport axonal du BDNF dans les motoneurones, améliorer l'activation de l'ERK dans les motoneurones, améliorer la formation et la préservation de la jonction neuromusculaire (NMJ), préserver la structure de la NMJ, améliorer la fonction de la NMJ, améliorer le taux d'innervation du tissu musculaire, améliorer la croissance axonale des motoneurones, réduire la dégénérescence axonale, réduire la dégénérescence axonale des motoneurones, améliorer la survie des cellules musculaires, améliorer le diamètre et la fonction des fibres musculaires ou réduire l'agrégation de SOD1.

6. Combinaison destinée à être utilisée selon la revendication 1, dans laquelle la combinaison réduit en outre la progression de la maladie pseudobulbaire, réduit la progression de l'atrophie des fibres musculaires et/ou améliore la contraction musculaire chez le sujet atteint de SLA.

7. Combinaison destinée à être utilisée selon l'une des revendications 1-6, dans laquelle la composition comprenant de la pridopidine ou un de ses sels pharmaceutiquement acceptables et la deuxième composition sont administrées quotidiennement, deux fois par jour, deux fois par semaine, trois fois par semaine ou plus d'une fois par jour.

8. Combinaison destinée à être utilisée selon l'une des revendications 1-7, dans laquelle la composition comprenant de la pridopidine ou un de ses sels pharmaceutiquement acceptables est administrée par voie orale ou par sonde gastrique.

9. Combinaison destinée à être utilisée selon l'une des revendications 1-8, dans laquelle la quantité administrée de pridopidine ou un de ses sels pharmaceutiquement acceptables est comprise entre 10 mg par jour et 135 mg par jour.

10. Combinaison destinée à être utilisée selon l'une des revendications 1-9, dans laquelle la pridopidine est du chlorhydrate de pridopidine.

11. Combinaison destinée à être utilisée selon l'une des revendications 1-10, dans laquelle la quantité administrée d'or nanocristallin CNM-Au8 est comprise entre 5 et 50 mg/jour et est administrée par voie orale.

12. Combinaison destinée à être utilisée selon l'une des revendications 1-11, dans laquelle l'administration de la deuxième composition précède l'administration de la composition comprenant de la pridopidine ou un de ses sels pharmaceutiquement acceptables ; ou
dans laquelle l'administration de la composition comprenant de la pridopidine ou un de ses sels pharmaceutiquement acceptables précède l'administration de la deuxième composition ; ou
dans laquelle la composition comprenant de la pridopidine ou un de ses sels pharmaceutiquement acceptables est administrée à titre adjuvant de la deuxième composition ; ou
dans laquelle la deuxième composition est administrée en complément de la composition comprenant de la pridopidine ou un de ses sels pharmaceutiquement acceptables.
